# EUROPEAN PATENT SPECIFICATION

(11) **EP 2 618 815 B1**
(45) Date of publication and mention of the grant of the patent: **15.11.2017**
(21) Application number: 11770892.5
(22) Date of filing: 24.08.2011
(51) Int. Cl.: A61K 9/08, A61K 9/107, A61K 9/20, A61K 9/48, A61K 31/352

(54) **PHARMACEUTICAL COMPOSITIONS FOR CALANOLIDES, THEIR DERIVATIVES AND ANALOGUES, AND PROCESS FOR PRODUCING THE SAME**
PHARMAZEUTISCHE ZUSAMMENSETZUNGEN FÜR CALANOLIDE, IHRE DERIVATE UND ANALOGA SOWIE VERFAHREN ZU IHRER HERSTELLUNG
COMPOSITIONS PHARMACEUTIQUES POUR CALANOLIDES, LEURS DÉRIVÉS ET ANALOGUES, ET PROCÉDÉ DE PRODUCTION ASSOCIÉ

(30) Priority: 22.09.2010 MY PI2010004411
(43) Date of publication of application: 31.07.2013
(73) Proprietor: Craun Research Sdn Bhd, 93055 Kuching, Sarawak (MY)
(72) Inventor: PHANG, Nyie Lin, Selangor (MY); ABDULLAH, Zaliha Christine, Sarawak (MY)
(74) Representative: Fairbairn, Angus Chisholm
(86) International application number: PCT/MY2011/000192
(87) International publication number: WO 2012/039596

(56) References cited:
- US-A1- 2002 012 680
- US-A1- 2008 038 335
- US-B1- 6 268 393
- HAI XUE ET AL: "Highly suppressing wild-type HIV-1 and Y181C mutant HIV-1 strains by 10-chloromethyl-11-demethyl-12-oxo-calanol ide A with druggable profile", JOURNAL OF MEDICINAL CHEMISTRY, AMERICAN CHEMICAL SOCIETY, US, vol. 53, no. 3, 11 February 2010 (2010-02-11), pages 1397-1401, XP002622243, ISSN: 0022-2623, DOI: 10.1021/JM901653E [retrieved on 2001-01-05]

## Description

### Technical Field of the Invention

The present invention relates to pharmaceutical compositions of calanolides, their derivatives and analogues, and process for producing the same having enhanced solubility and bioavailability.

More particularly it relates to pharmaceutical compositions of calanolides, their derivatives and analogues for oral or parenteral administration.

The invention further provides for a method of using the disclosed compositions for the treatment and prevention of retroviral diseases and mycobacterial infections in mammals, particularly in humans.

### Background of the Invention

The increasing incidence of infectious diseases necessitates continuing efforts to develop new drugs for managing and combating these diseases. Among those infectious diseases, human immunodeficiency virus (HIV) and tuberculosis (TB) are the leading cause of death.

Calanolides, their derivatives and analogues are a group of antiviral compounds as described in U.S. Pat Nos. 5,591,770, 5,859,049 and 6,268,393, which are incorporated by reference herein in their entireties. Of the calanolides, calanolide A is known for inhibiting the growth and replication of retrovirus, in particular but not limited to, HIV Type 1 (HIV-1). Calanolides and analogues thereof also demonstrate significant anti-mycobacterial activity in particular against Further publications include US 6268393, US 2008/038335, and US 2002/012680.

### Mycobacterium tuberculosis.

When calanolide A was administered in the form of soft gelatin capsules containing 100mg (+)-calanolide A formulated in an oil-based vehicle [Terri Creagh et al. Antimicrobial Agents and Chemotherapy, May 2001, p. 1379 - 1386] or as translucent soft gelatin capsules containing 100mg of (+)-calanolide A in a sesame oil-based vehicle [Eiznhamer et al. [HIV Clin Trials 2002; 3(6): 435 -450], both formulations showed poor bioavailability. In addition the above said studies also reported wide inter-subject variability in the pharmacokinetic parameters examined.

We have surprisingly found that the bioavailability of calanolide can be greatly improved by increasing the water solubility of calanolide, and this can be achieved by formulating calanolide with a combination of at least one solubility enhancer and at least one surfactant.

The compositions of the present invention are distinctly different from the teaching of Creagh & Eiznhamer in many ways. Firstly, the present invention provides pharmaceutical compositions having enhanced water solubility and bioavailability which have not been addressed in the disclosures of said studies. Therefore, the present invention is distinguishable from the formulations used in the said studies because none of the said studies addresses and overcomes the issue of poor bioavailability of calanolides.

Secondly, none of the formulations of the said studies disclosed, made obvious, or identified any pharmaceutical composition comprising a calanolide, a solubility enhancer and a surfactant having enhanced water solubility and bioavailability for calanolide.

Thus it is advantageous to have novel formulations of calanolides with enhanced bioavailability in order to reduce inter-patient and intra-patient variation in oral and parenteral bioavailability of calanolides.

Moreover, there is a need for and it is useful to have a pharmaceutical composition for the administration of calanolide, particularly for oral or parenteral administration, with enhanced bioavailability to reduce the side effects such as headache, nausea, belching, dizziness and liver toxicity associated with the use of calanolides as a medicament and further increase the therapeutic efficacy of calanolide compositions.

### Summary of the Invention

According to the present invention there is provided a pharmaceutical composition, a process for preparing same, and pharmaceutical compositions for use in treatment as defined in the appended independent claims. Further preferable features are defined in the appended dependent claims.
It is the primary object of the present invention to provide, in its broadest aspect, a pharmaceutical composition comprising:
a. a calanolide or mixture of calanolides;
b. one or more solubility enhancers, as defined in claim 1; and
c. one or more surfactants, as defined in claim 1.
It is another object of the present invention to provide pharmaceutical compositions having enhanced solubility and bioavailability for oral or parenteral administration wherein said compositions provide an increase in oral or parenteral bioavailability of calanolide as measured by Cₘₐₓ, of at least 30 percent and an increase of calanolide as measured by AUC₍₀₋₂₄₎, of at least 20 percent.
It is yet another object of the present invention to provide pharmaceutical compositions in the form of a liquid, semi-solid, or solid.
Still another object of the present invention is to provide pharmaceutical compositions comprising a calanolide in the unit dosage form of a soft capsule, a hard capsule, a tablet, an injection, an oral liquid preparation, a granule, or a pellet.
Another object of the present invention is to provide a process for preparing pharmaceutical compositions according to the first object of the invention.

It is yet another object of the present invention to provide a use of the pharmaceutical compositions in the preparation of a medicament for the treatment and prevention of retroviral diseases especially HIV-1 and mycobacterial diseases especially tuberculosis in mammals, particularly humans.

It is also to be understood that the foregoing general description is exemplary and explanatory, and is intended to provide for the explanation of the invention as claimed. Further objects of the invention, including advantages, novel features, and preferred embodiments of the present invention will be more apparent from the detailed description and examples that follow.

### Brief Description of the Figures

Table 1 shows solubility values for calanolide A.
Table 2 shows AUC₀₋₂₄ values of calanolide A in rats after oral administration of calanolide A.
Table 3 shows AUC₀₋₂₄ values of calanolide A in rats after intramuscular administration of calanolide A.
Figure 1 shows the plasma/serum concentration-time curves for calanolide A in rats following oral administration of pharmaceutical compositions according to the invention (Examples 1 and 2) in comparison with that of the control composition (Comparative Example A).
Figure 2 shows the plasma/serum concentration-time curves for calanolide A in rats following intramuscular administration of pharmaceutical composition according to the invention (Example 3) in comparison with that of the control composition (Comparative Example A).

### Detailed Description of the Invention

The present invention relates to pharmaceutical compositions for calanolides, their derivatives and analogues, and process for producing the same.

As used herein, the following terms have the following respective meanings:

"Pharmaceutical composition" is to be understood as defining composition which the individual components or ingredients are themselves pharmaceutically acceptable and are adapted to deliver a prescribed dosage of calanolide to a mammal, particularly, a human.

"Pharmaceutically acceptable" means having sufficiently low toxicity to be useable in a composition in the amount required.

"Water solubility" or "solubility" means the solubility of calanolide as measured in purified water at about 37°C, unless otherwise specified.

"Parenteral administration" means modes of administration other than enteral, topical and intravenous administrations, usually by injection, and includes, without limitation, intramuscular, intrathecal, intradermal, intraperitoneal, and subcutaneous administrations.

"Bioavailability" means the amount of a bioactive compound which reaches the blood circulation after its oral or parenteral administration.

"Oral bioavailability" means the total amount of calanolide absorbed into the plasma after a single oral administration.

"Parenteral bioavailability" means the total amount of calanolide absorbed into the plasma after a single parenteral administration, for example, intramuscular administration.

The present invention discloses a novel way to increase the oral or parenteral bioavailability of calanolide. More particularly, the present invention relates to pharmaceutical formulations, having enhanced solubility and bioavailability, which are useful for the oral or parenteral administration of calanolide.

We have found that when calanolide is combined with one or more solubility enhancers and one or more surfactants, a composition with enhanced solubility is obtained. This invention is based on our findings that oral or parenteral bioavailability of calanolide can be enhanced by combining calanolide with one or more solubility enhancers and one or more surfactants.

It must be understood that the pharmaceutical composition disclosed in the present invention is not a mere admixture but has properties different from the sum total of the properties of its ingredients. While not intending to be bound by this or any other theory, the solubility enhancer of this invention helps to increase the solubility of calanolide while the surfactant helps to improve wettability or dispersibility of the composition. This novel composition having enhanced solubility and bioavailability of the calanolide will result in greater absorption of the drug upon contact with gastrointestinal fluids.

Bioavailability of calanolide may be determined by measuring total systemic calanolide concentrations in a subject over time after oral or parenteral administration (e.g. intramuscular or subcutaneous) of said calanolide in the pharmaceutical composition of the present invention. The bioavailability is characterized by using the following parameters: the area under the blood-level curve (AUC), and/or the peak value of the blood-level maximum (Cₘₐₓ), both parameters are well known in the art.

As used herein, the term "subject" refers to an animal, preferably a mammal, including a rat or a human, who has been the object of treatment, observation or experiment.

AUC refers to the area under the curve that tracks the plasma concentration (ng/ml) of calanolide over a given time following oral or parenteral administration of the pharmaceutical composition of the present invention to a subject.

"AUC₀₋₂₄" refers to the area under the concentration-time curve from time zero to 24 hours.

Cₘₐₓ refers to the maximum plasma concentration of calanolide following oral or parenteral (e.g. intramuscular or subcutaneous) administration of the pharmaceutical composition of the present invention to a subject.

As used herein, and unless otherwise specified, the term "enhance" or "enhanced" when used in connection with the solubility of a calanolide, means an increased solubility of the calanolide of the present compositions as compared to the solubility of the same calanolide in a composition made according to the control composition (Comparative Example A). Specifically, the term "enhance" or "enhanced" means that, when the pharmaceutical composition of this invention is used, the solubility of a calanolide increases at least 20 percent more in comparison to that of the same calanolide in a control composition (Comparative Example A).

As used herein, the term "control composition" refers to a composition containing only calanolide A and sesame oil, without surfactant, as set forth in Comparative Example A.

Enhanced bioavailability of calanolide may be evidenced by an increase in AUC₀₋₂₄ and/or Cₘₐₓ for calanolide.

In one embodiment of the present invention, the term "enhanced bioavailability" of a pharmaceutical composition comprising calanolide is characterized by an increase in AU C₀₋₂₄ of calanolide at least 20 percent greater than that of the same calanolide in a control composition (Comparative Example A).

In another embodiment of the present invention, the term "enhanced bioavailability" of a pharmaceutical composition comprising calanolide is further characterized by an increase in Cₘₐₓ of calanolide at least 30 percent greater than that of the same calanolide in a control composition (Comparative Example A).

The pharmaceutical compositions of the present invention e.g. those in Examples 1 to 5 hereinafter, showed enhanced solubility as indicated by comparative solubility tests (as described in Example 9 hereinafter). The solubility tests were performed by using USP Paddle apparatus 2 as described under US Pharmacopoeia XXII, Dissolution Method, at 37°C, in purified water and at 50 rpm. The amount of dissolved calanolide was assayed by a high pressure liquid chromatography (HPLC) method.

The pharmaceutical compositions of the invention exhibit enhanced oral or parenteral bioavailability as demonstrated by bioavailability studies as set forth in Examples 10 and 11 hereinafter. The studies were performed in rats using high pressure liquid chromatographic (HPLC) methods to determine the level of the active ingredient absorbed in the blood. For example, the pharmaceutical compositions of Examples 1, 2 and 3 administered orally or parenterally to rats gave surprisingly high AUC₀₋₂₄ and Cₘₐₓ as determined by HPLC.

According to one aspect of this invention, it provides a pharmaceutical composition comprising calanolide characterized by an enhanced water solubility of calanolide.

In another aspect of this invention, it provides a pharmaceutical composition comprising calanolide characterized by enhanced oral or parenteral bioavailability of calanolide.
In yet another aspect, the invention provides a method of improving the bioavailability of calanolide in a subject (e.g., a mammal, particularly a human) comprising the step of: administering, by oral or parenteral route, to a subject in need of such treatment a therapeutically effective amount of a pharmaceutical composition of the present invention comprising a calanolide or mixture of calanolides; one or more solubility enhancers; and one or more surfactants. The said method enhances the bioavailability of calanolide administered by improving solubility of calanolide.
This method may be carried out in accordance with any of the techniques set forth therein, including employing, without limitation, any of the described dosage forms, particularly in the unit dosage forms set forth herein.

### Compositions

In a first aspect, of the present invention to provides, in its broadest aspect, a pharmaceutical composition comprising:
a. a calanolide or mixture of calanolides;
b. one or more solubility enhancers, as defined in claim 1; and
c. one or more surfactants, as defined in claim 1.

In accordance with one embodiment of the present invention, there is provided a pharmaceutical composition having enhanced solubility and bioavailability for oral or parenteral administration.
In accordance with another embodiment of the present invention, a preferred embodiment of the pharmaceutical composition comprises:
a. a calanolide or mixture of calanolides, in an amount of from about 0.5% to about 80% by weight of the total composition;
b. one or more solubility enhancers, in an amount of from about 0.5% to about 90% by weight of the total composition; and
c. one or more surfactants, in an amount of from about 0.1% to about 80% by weight of the total composition,
wherein the pharmaceutical composition has enhanced solubility and bioavailability for oral or parenteral administration.

In yet another embodiment of the invention, a more preferred pharmaceutical composition of the invention comprises:
a. a calanolide or mixture of calanolides, in an amount of from about 5% to about 50% by weight of the total composition;
b. one or more solubility enhancers, in an amount of from about 10% to about 60% by weight of the total composition; and
c. one or more surfactants, in an amount of from about 5% to about 50% by weight of the total composition,
wherein the pharmaceutical composition has enhanced solubility and bioavailability for oral or parenteral administration.

In accordance with yet another embodiment of the present invention, the most preferred pharmaceutical composition of the invention comprises:
a. a calanolide or mixture of calanolides, in an amount of from about 10% to about 35% by weight of the total composition;
b. one or more solubility enhancers, in an amount of from about 25% to about 50% by weight of the total composition; and
c. one or more surfactants, in an amount of from about 20% to about 40% by weight of the total composition,
wherein the pharmaceutical composition has enhanced solubility and bioavailability for oral or parenteral administration.

In still another embodiment of the present invention the pharmaceutical compositions can take the form of a liquid, semi-solid, or solid.

When pharmaceutical compositions in the form of solid are desired, solubility enhancers and surfactants in the form of solid at room temperature can be used. Examples of solubility enhancers in the form of solid are: beta cyclodextrin, polyethylene glycol 4000 (PEG 4000) and polyvinyl pyrrolidone K-30. Examples of solid surfactants are: poloxamer 338, sodium dioctylsulfosuccinade, and soy lecithin. Similarly, when the liquid or semi-solid pharmaceutical compositions are desired, the solubility enhancers and surfactants in the form of liquid or semi-solid at room temperature can be used. Examples of liquid or semi-solid solubility enhancers are: polyethylene glycol 200, N-methylpyrrolidone and sesame oil. Examples of liquid or semi-solid surfactants are: PEG-40 hydrogenated castor oil, PEG-35 castor oil and polysorbate 80.

### Calanolides

The first essential component of the pharmaceutical compositions of the present invention comprises a calanolide or mixture of calanolides as an active ingredient.
It should be understood that in this specification the terms "calanolide" or "calanolides" refer to any of the several calanolides of formulae I or II (as described in U.S. Pat Nos. 5,591,770 and 5,859,049, to Boyd et al. 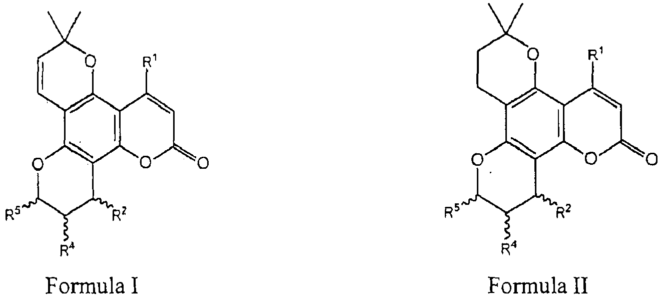 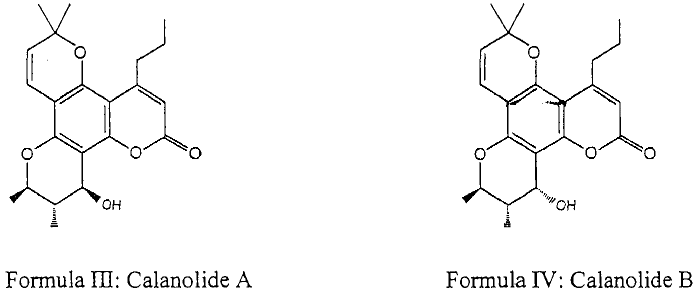 wherein
R¹ is C₁ - C₆ alkyl or aryl; R² is 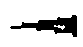 OH, 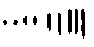 OH, 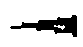 OR³, 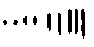 OR³, 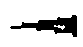 O₂ CR³, 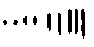 O₂CR³, 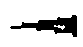 O₃SR³, or 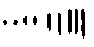 O₃SR³, wherein R³ is C₁ - C₆ alkyl or aryl; R⁴ and R⁵ are the same or different and are each 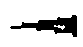 CH₃ or 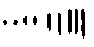 CH_{3.} While the aryl group maybe any suitable aryl substituent, the aryl is preferably a C₆ - C₁₄ ring structure, most preferably phenyl.

In addition, the "calanolide" or "calanolides" described herein may be present either substantially in the form of one optically pure enantiomer or as a mixture, racemic or otherwise, of enantiomers.

The "calanolide" or "calanolides" described herein may also be present as its derivatives, pharmaceutically acceptable salts, esters, amides, prodrugs, active metabolites, isomers, or analogues, provided that the derivatives, pharmaceutically-acceptable salts, esters, amides, prodrugs, active metabolites, isomers, or analogues are pharmacologically active and pharmaceutically acceptable.

As used herein, "pharmaceutically acceptable salts" refers to derivatives of calanolide wherein it is modified by making acid or base salts thereof. Examples of pharmaceutically acceptable salts include, but are not limited to, salts of organic or inorganic bases, such as: sodium, potassium, calcium, magnesium, ammonium, diethylamine, triethylamine, N-methyl-D-glucamine, arginine, lysine, and the like, and salts of organic or inorganic acids, such as hydrochloride, hydrobromide, tartrate, mesylate, acetate, maleate, oxalate, lactate, and the like.
The pharmaceutically acceptable salts of calanolide can be synthesized from the parent compound which contains a basic or acidic moiety by conventional chemical methods. Generally, such salts can be prepared by reacting the free acid or base forms of calanolide with a stoichiometric amount of the appropriate base or acid in water or in an organic solvent, or in a mixture of the two. Generally, non-aqueous media like ethanol, isopropyl alcohol, or ethyl acetate are preferred. Additional examples of pharmaceutically acceptable salts and their methods of preparation and use are presented in Pharmaceutical Salts: Properties, Selection, and Use - Second Revised Edition, (2002).

Preparation of esters involves transformation of a carboxylic acid group via a conventional esterification reaction involving nucleophilic attack of an RO⁻ moiety at the carbonyl carbon. Esterification may also be carried out by reaction of a hydroxyl group with an esterification reagent such as an acid chloride. Amides may be prepared from esters, using suitable amine reactants, or they may be prepared from anhydride or an acid chloride by reacting with ammonia or a lower alkyl amine. Prodrugs and active metabolites may also be prepared using techniques known to those skilled in the art or described in the pertinent literature. Prodrugs are typically prepared by covalent attachment of a moiety that results in a compound that is therapeutically inactive until modified by an individual's metabolic system.
Other derivatives and analogues of calanolides may be prepared using standard techniques known to those skilled in the art of synthetic organic chemistry, or may be deduced by reference to the pertinent literature.
Although calanolide A and calanolide B as shown in Formula III and IV respectively, and their analogues and derivatives of calanolide can be used as the calanolide component of the present invention, calanolide A is most preferred since its effectiveness and pharmacological properties are most established in the art.

### Particle size of calanolide

In one embodiment of the present invention, the calanolide particles can be present in any suitable form, for example, in a crystalline form, semi-crystalline form, amorphous form, semi-amorphous form, or combinations thereof.

In another embodiment of the present invention, the calanolide is present in the pharmaceutical compositions in the form of a dissolved liquid, a dispersed solid, or mixtures thereof.

In one aspect of the embodiment, the calanolide is completely dissolved in the pharmaceutical composition and takes the form of a liquid.

In another aspect, the calanolide is present as dispersed solid with a particle size of less than 1000 micron, more preferably in the form of solid with particle size of less than 50 micron, and most preferably, less than 0.1 micron.

In yet another aspect, the calanolide is present as a mixture of liquid and dispersed solid, wherein the said dispersed solid has a particle size of less than 1000 micron, more preferably in the form of solid with particle size of less than 50 micron, and most preferably, less than 0.1 micron.

As used herein, particle size refers to average particle size as measured by conventional particle size measuring techniques well known in the art, such as sedimentation field flow fractionation, photon correlation spectroscopy, or disk centrifugation.

The particle size of the calanolide present in the pharmaceutical composition of the present invention can be reduced before said calanolide is added to the said composition by applying mechanical means in the presence of grinding media to reduce the particle size to the required range. The mechanical means applied to reduce the particle size of the calanolide conveniently can take the form of a dispersion mill, for example: a ball mill or a vibratory mill. The calanolide of reduced particle size can then be used as starting raw material for the pharmaceutical compositions of the present invention.

Optionally, the particle size of the calanolide can be reduced by first dispersing the calanolide in a liquid solubility enhancer and followed by applying mechanical means in the presence of grinding media to reduce the particle size to the required range.

### Solubility enbancer

The second component of the pharmaceutical compositions of the present invention comprises one or more solubility enhancers selected from the group consisting of oils, pyrrolidone derivatives, polyglycols, aliphatic amides, polyethers, alcohols, esters of alcohols, dimethyl sulfoxide, cyclodextrins, and combinations thereof.

The solubility enhancers used for the pharmaceutical compositions of the present invention are preferably having an accepted status, e.g. GRAS substances (Generally Regarded As Safe Food Additives - GRAS substances, Food Drug Cosmetic Law Reports, Chicago (1994), Food Additive Database under FDA).

In another embodiment of the invention, pharmaceutically acceptable solubility enhancers are preferably to be selected for the compositions of the present invention. A "pharmaceutically acceptable solubility enhancer" as used herein, generally refers to a solubility enhancer which is compatible with other ingredients of the pharmaceutical compositions and suitable for use in contact with the tissues of human beings and animals without excessive toxicity, irritation, allergic response, or other problem or complication, commensurate with a reasonable benefit/risk ratio.

In one embodiment of the present invention, the oils suitable for use as the solubility enhancer component in the pharmaceutical compositions of the present invention may be selected from vegetable oils, animal oils, triglycerides, diglycerides, monoglycerides, fatty acids, and mixtures thereof.

Examples of vegetable oils suitable for use in the invention include, but not limited to, almond oil, apricot kernel oil, babassu oil, borage oil, blackcurrant seed oil, canola oil, castor oil, coconut oil, corn oil, cotton seed oil, evening primrose oil, grape seed oil, groundnut oil, mustard seed oil, olive oil, palm oil, palm kernel oil, peanut oil, grape seed oil, rapeseed oil, safflower seed oil, sesame oil, soybean oil, sunflower seed oil, and mixtures thereof.

Examples of animal oils include, but not limited to, fish oil, eel oil, fish liver oil, shark oil, mink oil, and mixtures thereof.

Triglycerides are glycerides formed from a single molecule of glycerol esterified with three fatty acids. Examples of triglycerides suitable for use in the present invention include: Caprylic / capric triglyceride (Miglyol 810, Sasol); caprylic/capric/linoleic triglyceride (Miglyol 818, Sasol), Glyceryl tricaprate (Captex 1000, Abitec), Glyceryl trimyristate (Dynasan 114, Huls), Glyceryl tripalmitate (Dynasan 116, Huls), Glyceryl tristearate (Dynasan 118, Huls), Glyceryl tricaprylate/caprate/laurate (Captex 350 , Abitec), Super Refined Corn Oil (CRODA), Cottonseed Oil, Super Refined Cottonseed Oil (CRODA), Super Refined Safflower Oil (CRODA), and Super Refined Sesame Oil (CRODA).

Monoglycerides are glycerides formed from a single molecule of glycerol esterified with one fatty acid. Diglycerides are glycerides formed from a single molecule of glycerol esterified with two fatty acids. Examples of monoglycerides and diglycerides suitable for use in the pharmaceutical compositions of the present invention include, but not limited to, glyceryl monocaprate, glyceryl monocaprylate, glyceryl monostearate, glyceryl monooleate, glyceryl dicaprylate, glyceryl dilaurate, and mixtures thereof.

Fatty acids are aliphatic monocarboxylic acids derived from, or contained in esterified form in, an animal or vegetable fat, oil, or wax. Fatty acids can be saturated and unsaturated. Examples of fatty acids suitable for use in the pharmaceutical compositions of the present invention include, but not limited to, butyric acid (C4), caproic acid (C6), caprylic acid (C8), capric acid (C10), lauric acid (C12), myristic acid (C14), palmitic acid (C16), stearic acid (C18), arachidic acid (C20), behenic acid (C22), α-linolenic acid, linoleic acid, alpha-linolenic acid, gamma-linolenic acid, eicosapentaenoic acid (EPA), docosahexaenoic acid (DHA), docosapentaenoic acid (DPA), arachidonic acid, and mixtures thereof.

One skilled in the art will also appreciate that other oils may be used as solubility enhancers in the pharmaceutical compositions of the present invention include, but are not limited to, synthetic and semi-synthetic monoglycerides, diglycerides, triglycerides, and mixtures thereof.

In another embodiment of the present invention, the solubility enhancer suitable for use in the pharmaceutical compositions of the present invention comprises of pyrrolidone derivatives. Examples of suitable pyrrolidone derivatives, include but not limited to, 2-pyrrolidone, N-methyl-2-pyrrolidone, N-ethyl-2-pyrrolidone, N-propy-2-pyrrolidone, N-isopropyl-2-pyrrolidone, N-butyl-2-pyrrolidone, and polyvinylpyrrolidones (PVP) having an average molecular weight of 2000 to 1200000 including polyvinylpyrrolidone K-30, polyvinylpyrrolidone K-90, and mixtures thereof.

In another further embodiment of the present invention, the solubility enhancer suitable for use in the pharmaceutical compositions of the present invention comprises of polyglycols. Examples of suitable polyglycols include, but not limited to, polyethylene glycols of different molecular weights, particularly those with molecular weights between 200 and 20000, and mixtures thereof.

When a liquid is desired for the final composition or a liquid is to be used to fill soft capsules, preferably soft gelatin capsules, the preferred molecular weight range of polyethylene glycol is from about 200 to about 600 with polyethylene glycols 400 being especially preferred. When a semi-solid or solid is preferred, especially for filling a hard capsule, preferably a hard gelatin capsule, a preferred polyethylene glycol molecular weight is about 3350.

In yet another embodiment of the present invention, the solubility enhancer suitable for use in the pharmaceutical compositions of the present invention comprises of aliphatic amides. Examples of suitable aliphatic amides include, but not limited to, dimethyl formamide, dimethyl acetamide, and mixtures thereof.

In yet another further embodiment of the present invention, the solubility enhancer suitable for use in the pharmaceutical compositions of the present invention comprises of polyethers. Examples of suitable polyethers include, but not limited to, isosorbide dimethyl ether, diethylene glycol monoethyl ether (available from Gattefosse under the trade name Transcutol), tetrahydrofurfuryl alcohol PEG ether (Glycofurol, available from BASF under the trade name of Tetraglycol), tetrahydrofuran, 1,4-dioxane, and mixtures thereof.

In another embodiment of the present invention, the solubility enhancer suitable for use in the pharmaceutical compositions of the present invention comprises of alcohols. Examples of alcohols include, but not limited to, ethanol, isopropyl alcohol, glycerol, butanol, benzyl alcohol, ethylene glycol, propylene glycol, pentaerythritol, sorbitol, mannitol, and mixtures thereof.

In a further embodiment of the present invention, the solubility enhancer suitable for use in the pharmaceutical compositions of the present invention comprises of esters of alcohols. Examples of suitable esters of alcohols include, but not limited to, isopropyl myristate, isopropyl oleate, octyl palmitate, glycerol monostearate, pentaerythritol distearate, propylene glycol monoacetate, propylene glycol monolaurate, propylene glycol dilaurate, propylene glycol hydroxystearate, propylene glycol isostearate , propylene glycol ricinoleate, propylene glycol diacetate, propylene glycol monocaprylate, propylene glycol dicaprylate, propylene glycol dicaprate, propylene glycol dicaprylate/dicaprate, triacetin (glycerin triacetate), diacetin (glycerin diacetate), D-alpha tocopheryl polyethylene glycol 1000 succinate, and mixtures thereof.
In another embodiment of the present invention, the solubility enhancer suitable for use in the pharmaceutical compositions of the present invention comprises dimethyl sulfoxide.
In yet another embodiment of the present invention, the solubility enhancer suitable for use in the pharmaceutical compositions of the present invention comprises of cyclodextrins. Examples of suitable cyclodextrins include, but not limited to, alpha-cyclodextrin, beta-cyclodextrin, gamma-cyclodextrin, hydroxypropyl alpha-cyclodextrin, methylated alpha-cyclodextrin, methylated beta-cyclodextrin, hydroxyethyl beta-cyclodextrin, hydroxypropyl beta-cyclodextrin, hydroxypropyl gamma-cyclodextrin, and methylated gamma-cyclodextrin, and mixtures thereof. A particularly preferred cyclodextrin is hydroxypropyl-beta-cyclodextrin. According to the invention, the solubility enhancer is selected from the group consisting of sesame oil, olive oil, glyceryl tricaprylate/caprate, glyceryl tricaprylate/caprate/linoleate, glyceryl monocaprate, glyceryl monocaprylate, glyceryl monostearate, glyceryl monooleate, glyceryl dicaprylate, glyceryl dilaurate, isopropyl myristate, isopropyl oleate, 2-pyrrolidone, N-methyl-2-pyrrolidone, PEG 200, PEG 300, PEG 400, beta-cyclodextrin, hydroxypropyl beta-cyclodextrin, and mixtures thereof.
In the most preferred embodiment of the invention, the solubility enhancer is selected from the group consisting of sesame oil, glyceryl tricaprylate/caprate, 2-pyrrolidone, N-methyl-2-pyrrolidone, beta-cyclodextrin, hydroxypropyl beta-cyclodextrin, and mixtures thereof.

### Surfactants

The third component of the pharmaceutical compositions in accordance with the invention comprises one or more surfactants.

The surfactant in the invention can be selected from the group consisting of nonionic, anionic, cationic, and zwitterionic surfactants, and combinations thereof.

Again, in general, surfactants used for the pharmaceutical compositions of the present invention are preferably having an accepted status, e.g. GRAS substances (Generally Regarded As Safe Food Additives - GRAS substances, Food Drug Cosmetic Law Reports, Chicago (1994), Food Additive Database under FDA).

Alternatively, pharmaceutically acceptable surfactants are preferred to be selected for the compositions of the present invention. A "pharmaceutically acceptable surfactant" as used herein, generally refers to a surfactant which is compatible with other ingredients of the pharmaceutical compositions and is suitable for use in contact with the tissues of human beings and animals without excessive toxicity, irritation, allergic response, or other problem or complication, commensurate with a reasonable benefit/risk ratio. It should be emphasized that the invention is not limited to the surfactants described below, which show representative, but not exclusive, lists of available surfactants. In addition, refined, distilled, or fractionated surfactants, purified fractions thereof, or re-esterified fractions, are also within the scope of the invention, although not specifically listed below.

In the pharmaceutical composition of the present invention, either any one of the above defined surfactants alone or a mixture of two or more surfactants can be used as the surfactant component.

In one embodiment of the present invention, the preferred surfactants for use as surfactant component are nonionic surfactants. Examples of suitable nonionic surfactants are as follows:
(i). Reaction products of natural or hydrogenated vegetable oils and ethylene glycol.

| **Ethylene glycol - Vegetable Oil Ester Surfactants** | |
|---|---|
| **COMPOUND** | **COMMERCIAL PRODUCT (supplier)** |
| PEG-35 castor oil | Cremophor EL (BASF) |
| PEG-10 hydrogenated castor oil | Nikkol HCO-10 (Nikko) |
| PEG-60 hydrogenated castor oil | Nikkol HCO-60 (Nikko) |
| PEG-40 hydrogenated castor oil | Cremophor RH 40 (BASF) |
| PEG-60 hydrogenated castor oil | Cremophor RH 60 (BASF) |
| PEG-100 hydrogenated castor oil | Nikkol HCO-100 (Nikko) |

Especially preferred products of this class for use in the pharmaceutical compositions of the present invention are PEG-35 castor oil and PEG-40 hydrogenated castor oil.
(ii) Polyoxyethylene sorbitan fatty acid esters which are commercially available under the trade name TWEEN.

| **Polyoxyethylene-Sorbitan Fatty Acid Ester Surfactants** | |
|---|---|
| **COMPOUND** | **COMMERCIAL PRODUCT (supplier)** |
| PEG-20 sorbitan monolaurate | Tween-20 (CRODA) |
| PEG-20 sorbitan monopalmitate | Tween-40 (CRODA) |
| PEG-20 sorbitan monostearate | Tween-60 (CRODA) |
| PEG-20 sorbitan monooleate | Tween-80 (CRODA) |
| PEG-20 sorbitan trioleate | Tween-85 (CRODA) |
| PEG-20 sorbitan tristearate | Tween 65 (CRODA) |
| PEG-4 sorbitan monolaurate | Tween-21 (CRODA) |
| PEG-4 sorbitan monostearate | Tween-61 (CRODA) |
| PEG-5 sorbitan monooleate | Tween-81 (CRODA) |

Especially preferred products of this class for use in the pharmaceutical compositions of the present invention are PEG-20 sorbitan monolaurate, PEG-20 sorbitan monopalmitate and PEG-20 sorbitan monooleate.
(iii) Polyoxyethylene fatty acid esters, for example polyoxyethylene stearic acid esters of the type known and commercially available under the trade name MYRJ (CRODA) as well as polyoxyethylene fatty acid esters known and commercially available under the trade name Cithrol (CRODA).

| **PEG-Fatty Acid Ester Surfactants** | |
|---|---|
| **COMPOUND** | **COMMERCIAL PRODUCT (supplier)** |
| PEG-8 distearate | Cithrol 4DS (CRODA) |
| PEG-2 stearate | Cithrol DE GMS (CRODA) |
| PEG-8 laurate | Cithrol 4 ML (CRODA) |
| PEG-8 dioleate | Cithrol 4DO (CRODA) |
| PEG-12 stearate | Cithrol 6MS (CRODA) |
| Polyoxyethylene (40) stearate | Myrj S40 (CRODA) |
| Polyoxyethylene (100) stearate | Myrj S100 (CRODA) |

A preferred product of this class for use in the pharmaceutical compositions of the present invention is polyoxyethylene (40) stearate.
(iv) Trans-esterification products of natural vegetable oil triglycerides and polyalkylene polyols. They include trans-esterification products of various natural (e.g. non-hydrogenated) vegetable oils for example, maize oil, kernel oil, almond oil, ground nut oil, olive oil and palm oil and mixtures thereof with polyethylene glycols, in particular polyethylene glycols having an average molecular weight of from 200 to 800. Various forms of trans-esterification product of the class defined are known and commercially available under the trade name LABRAFIL, which can be obtained, for example, from Gattefossé, Saint-Priest Cedex, France.

| **Alcohol-Oil Trans-esterified Surfactants** | |
|---|---|
| **COMPOUND** | **COMMERCIAL PRODUCT (supplier)** |
| PEG-6 corn oil | Labrafil M2125 CS (Gattefosse) |
| PEG-6 almond oil | Labrafil M1966 CS (Gattefosse) |
| PEG-6 apricot kernel oil | Labrafil M1944 CS (Gattefosse). |
| PEG-6 olive oil | Labrafil M1980 CS (Gattefosse) |
| PEG-6 peanut oil | Labrafil M1969 CS (Gattefosse) |
| PEG-6 palm kernel oil | Labrafil M2130 CS (Gattefosse) |

Especially preferred products of this class for use in the pharmaceutical compositions of the present invention are PEG-6 apricot kernel oil and PEG-6 corn oil.
(v) Sorbitan fatty acid esters e.g. of the type known and commercially available under the trade name SPAN.

| **Sorbitan Fatty Acid Ester Surfactants** | |
|---|---|
| **COMPOUND** | **COMMERCIAL PRODUCT (supplier)** |
| Sorbitan oleate | Span® 80 (CRODA) |
| Sorbitan sesquioleate | Span® 43 (CRODA) |
| Sorbitan isostearate | Span® 6 (CRODA) |
| Sorbitan tristearate | Span® 65 (CRODA) |

Especially preferred products of this class for use in the pharmaceutical compositions of the present invention are sorbitan oleate and sorbitan tristearate.
(vi) Polyoxyethylene-polyoxypropylene block co-polymers, e.g. of the type known and commercially available under the trade name POLOXAMER.

| **POE-POP Block Copolymers Surfactants** | |
|---|---|
| **COMPOUND** | **COMMERCIAL PRODUCT (supplier)** |
| Poloxamer 338 | Pluronic® F108 (BASF) |
| Poloxamer 407 | Pluronic® F127 (BASF) |
| Poloxamer 188 | Pluronic® F68 (BASF) |
| Poloxamer 237 | Pluronic® F87 (BASF) |
| Poloxamer 124 | Pluronic® L44 (BASF) |

A preferred product of this class for use in the pharmaceutical compositions of the present invention is the product POLOXAMER 188.
(vii) Sucrose fatty acid esters, for example, the type known and commercially available under the trade names of Crodesta and Isolan.

| **Glucose Esters Surfactants** | |
|---|---|
| **COMPOUND** | **COMMERCIAL PRODUCT (supplier)** |
| Methyl glucose dioleate | Isolan® DO (Goldsmith) |
| Methyl glucose isostearate | Isolan® IS (Goldsmith) |
| Sucrose distearate | Crodesta F-10 (CRODA) |
| Sucrose monostearate | Crodesta F-160 (CRODA) |
| Sucrose monopalmitate | SUCRO ESTER 15 (Gattefosse) |

A preferred product of this class for use in the pharmaceutical compositions of the present invention is sucrose distearate.

In another embodiment of the present invention, surfactants for use as surfactant component are ionic surfactants. The pharmaceutically acceptable ionic surfactants can be selected from the group comprises of cationic surfactants, anionic surfactants, zwitterionic surfactants, and combinations thereof. Examples of ionic surfactants selected from the group are as follows:-
i) Suitable anionic surfactants are listed below.

| **Anionic Surfactants** | |
|---|---|
| **COMPOUND** | **COMMERCIAL PRODUCT (supplier)** |
| Fatty Acid Salts | |
| Sodium caproate | Sodium hexanoate (Sigma) |
| Sodium caprylate | Sodium octanoate (Sigma) |
| Sodium caprate | Sodium decanoate (Sigma) |
| Sodium laurate | Sodium dodecanoate (Sigma) |
| Sodium oleate | Sodium oleate (Sigma) |
| Sodium palmitate | Sodium palmitate (Sigma) |
| Sodium lauryl sulfate | Texapon K 12GPH (Cognis) |
| Sodium dioctyl sulfosuccinate | Sodium Docusate (Cytec) |

| Bile Salts | |
|---|---|
| Sodium cholate | Sodium cholate hydrate (Sigma) |
| Sodium glycodeoxycholate | Sodium glycodeoxycholate (Sigma) |
| Sodium deoxycholate | Sodium deoxycholate monohydrate (Sigma) |

A preferred product of this class for use in the pharmaceutical compositions of the present invention is sodium oleate, sodium palmitate, sodium lauryl sulfate, sodium dioctyl sulfosuccinate, sodium cholate, and mixtures thereof.
ii) Suitable cationic surfactants are listed below.

| **Cationic Surfactants** |
|---|
| **COMPOUND** |
| Lauroyl carnitine |
| Palmitoyl carnitine |
| Myristoyl carnitine |
| Cetyl trimethyl ammonium bromide |
| Dodecyl ammonium chloride |
| Betaines (Trialkylglycine) |
| Lauryl betaine |
| Alkyl benzyldimethylammonium salts (benzalkonium chloride) |

A preferred product of this class for use in the pharmaceutical compositions of the present invention is lauroyl carnitine, cetyl trimethyl ammonium bromide, dodecyl ammonium chloride, betaines, benzalkonium chloride and mixtures thereof.
iii) Suitable zwitterionic surfactants are listed below. **Zwitterionic Surfactants**

| **COMPOUND** | **COMMERCIAL PRODUCT (supplier)** |
|---|---|
| Phostiholipids | |
| Soy lecithin | Epikuron 200 (Lucas Meyer) |
| Hydroxylated lecithin | Phospholipon 90H and 100H (Natterman) |
| Deoiled hydrolyzed lecithin | Emultop™ (Cargill) |
| Deoiled lecithin | Epikuron™ 100 P (Cargill) |
| Lysophosphatidylcholine | Lysophosphatidylcholine (Cargill) |
| Deoiled lecithin | Ultralec® (ADM) |
| Hydrolyzed lecithin | Adlec™ E (ADM) |
| Phosphatidylcholine | 99% soy phosphatidylcholine (Avanti Polar Lipids) |
| Hydrogenated lecithin | Epikuron H 100 (Lucas Meyer) |

Especially preferred products of this class for use in the pharmaceutical compositions of the present invention are soy lecithin, hydroxylated lecithin, lysophosphatidylcholine, phosphatidylcholine, hydrogenated lecithin, and mixtures thereof. The surfactants used according to the invention are shown below. Mixtures of these surfactants can also be used.

| **COMPOUND** | **TRADE NAME (supplier)** |
|---|---|
| Polyoxyethylene (20) sorbitan monolaurate | Tween 20 (CRODA) |
| Polyoxyethylene (20) sorbitan monopalmitate | Tween 40 (CRODA) |
| Polyoxyethylene (20) sorbitan monostearate | Tween 60 (CRODA) |
| Polyoxyethylene (20) sorbitan monooleate | Tween 80 (CRODA) |
| PEG-40 hydrogenated castor oil | Cremophor RH40 (BASF) |
| Polyoxyethylene (35) castor oil | Cremophor EL (BASF) |
| PEG-6 apricot kernel oil | Labrafil M1944 CS (Gattefosse) |

### Process for Producing Pharmaceutical Compositions

The present invention also provides for a process for producing pharmaceutical compositions comprising the steps of:
i. mixing a calanolide with one or more solubility enhancers at temperature between 20°C to 65°C; and
ii. mixing one or more surfactants to the resulting mixture obtained in step (i) to form a homogenous mixture.
wherein the mixing process in step (i) can further comprise an additional step of grinding the powder mixture in a dispersion mill for 1 to 40 hours.

In another embodiment, the invention provides a process of producing pharmaceutical compositions of the present invention comprising the steps of:
i) mixing a calanolide or mixture of calanolides in an amount of from about 0.5% to about 80% by weight of the total composition with one or more solubility enhancers in an amount of from about 0.5% to about 90% by weight of the total composition, at temperature between 20°C to 65°C;
ii) mixing the resulting mixture obtained in step (i) with one or more surfactants in an amount of from about 0.1 % to about 80% to form a homogenous mixture;
wherein the mixing process in step (i) can further comprise an additional step of grinding the powder mixture in a dispersion mill for 1 to 40 hours.

In yet another embodiment, the invention provides a process of preparing the pharmaceutical compositions comprising the steps of:
i) mixing a calanolide or mixture of calanolides in an amount of from about 5% to about 50% by weight of the total composition with one or more solubility enhancers in an amount of from about 10% to about 60% by weight of the total composition, at temperature between 20°C to 65°C;
ii) mixing the resulting mixture obtained in step (i) with one or more surfactants in an amount of from about 5 % to about 50% to form a homogenous mixture; and
wherein the mixing process in step (i) can further comprise an additional step of grinding the powder mixture in a dispersion mill for 1 to 40 hours.

In still yet another embodiment, the present invention provides a process of preparing the pharmaceutical compositions comprising the steps of:
i) mixing a calanolide or mixture of calanolides in an amount of from about 10% to about 35% by weight of the total composition with one or more solubility enhancers in an amount of from about 25% to about 50% by weight of the total composition, at temperature between 20°C to 65°C;
ii) mixing the resulting mixture obtained in step (i) with one or more surfactants in an amount of from about 20 % to about 40% to form a homogenous mixture;
wherein the mixing process in step (i) can further comprise an additional step of grinding the powder mixture in a dispersion mill for 1 to 40 hours.

In one variation of the embodiment, where the pharmaceutical composition comprises at least one solubility enhancer present in the form of solid, such as cyclodextrins or polyvinylpyrrolidones, the said mixing process in step (i) can further comprise an additional step of grinding the powder mixture in a dispersion mill for example a ball mill or air-jet mill for 1 to 40 hours.

### Unit Dosage Form

In yet another aspect of the present invention, the pharmaceutical compositions may conveniently be presented in unit dosage forms and may be prepared by any of the methods well known in the art.

The pharmaceutical compositions may be formulated in conventional manner, if desired, with further pharmaceutically acceptable excipients, into forms suitable for oral or parenteral administration. Suitable formulation is dependent upon the route of administration selected.

In the present context the term "pharmaceutically acceptable excipient" is intended to denote any material, which is inert and non-toxic in the sense that it substantially does not have any therapeutic and/or prophylactic effect *per se.* Such excipient(s) may be added with the purpose of making it possible to obtain a pharmaceutical composition which has acceptable technical properties.

Examples of suitable pharmaceutically acceptable excipients for use in a dosage form according to the invention include fillers, diluents, glidants, disintegrants, binders, lubricants, etc. or mixture thereof. Other pharmaceutically acceptable excipients for suitable use are e.g. acidifying agents, alkalizing agents, preservatives, antioxidants, buffering agents, chelating agents, coloring agents, complexing agents, flavors and perfumes, humectants, and sweetening agents.

Specific examples of suitable fillers, diluents and/or binders include lactose, microcrystalline cellulose, hydroxypropylcellulose, hydroxypropyl methylcellulose (HPMC), sodium carboxymethylcellulose, microcrystalline cellulose, sucrose, sorbitol, mannitol, dextrins, maltodextrins, starches or modified starches (including potato starch, maize starch and rice starch), povidone, guar gum, dicalcium phosphate, tricalcium phosphate, calcium sulfate, calcium carbonate, sodium alginate, etc.

Specific examples of disintegrants are croscarmellose sodium, crospovidone, sodium starch glycolate, starch, and pregelatinized starch.

Glidants and lubricants may also be included in the composition. Examples include stearic acid, magnesium stearate, talc, and colloidal silica.

Most of these excipients are described in detail in the "Handbook of Pharmaceutical Excipients" (6^{th} Edition, published jointly by the American Pharmacists Association and the Pharmaceutical Press, publications department of the Royal Pharmaceutical Society of Great Britain, the Pharmaceutical Press, 2009), the disclosure of which is incorporated by reference herein in its entirety.

In one embodiment, the pharmaceutical compositions may be formulated in conventional manner into solid dosage forms for oral administration. Examples of preferred solid dosage forms are: granules, pellets, powders, tablets, and hard capsules.

In another embodiment, the pharmaceutical compositions may be formulated in conventional manner into liquids or semi-solids for oral administration. Examples of preferred liquid or semi-solid dosage forms are: oral drinking liquids, soft gelatin capsules, soft capsules with non-gelatin shells, hard gelatin capsules, and hard capsules with non-gelatin shells.

In yet another embodiment, the pharmaceutical compositions may be formulated in conventional manner into solutions or suspensions for parenteral administration. Examples of preferred dosage forms in solutions or suspensions are: solutions or suspensions for intramuscular, subcutaneous, intraperitoneal, and/or intradermal administrations.

Methods of making oral or parenteral dosage forms are known in the art, and such methods can be employed in the present invention.

### Softgels

When the pharmaceutical composition of the present invention is prepared in the form of soft capsules; the composition may be encapsulated in a gelatin, starch, carrageenan, or cellulose soft shell which contains any conventional plasticizer. Suitable plasticizers are: glycerin, sorbitol, propylene glycol, polyethylene glycol, and mixtures of these plasticizers. Additionally the capsule material may contain additives like preservatives, opacifying agents, colouring and flavouring agents.

The composition is incorporated into the soft capsule shell by conventional procedures as described in detail in "The Theory and Practice of Industrial Pharmacy" (by Leon Lachman et al., Lea and Febiger, Philadelphia, USA, 2nd edition, 1976), the disclosure of which is incorporated by reference herein in its entirety.

### Hard shell capsules

The pharmaceutical composition of the present invention, in liquid or semi-solid, may be prepared in the form of hard shell capsules. The composition may be encapsulated in a gelatin, starch, carrageenan, or cellulose hard shell which contains any conventional plasticizer. Suitable plasticizers are: glycerin, sorbitol, propylene glycol, polyethylene glycol, and mixtures of these plasticizers. Additionally the capsule material may contain additives like preservatives, opacifying agents, coloring and flavouring agents. The shells are generally supplied in two interlocking portions, one of which is filled with the composition of the present invention and the other of which is placed over the filled shell portion by acting as a cap. The two portions can then be sealed by any convenient means such as by gelatin banding or by LICAPS system marketed by Capsulgel (The word LICAPS is a trade mark of Capsulgel).

The pharmaceutical composition of the present invention, in solid form, may also be prepared in the form of hard shell capsules. The hard capsules can contain the active ingredient in admixture with fillers such as lactose, binders such as starches, and/or lubricants such as talc or magnesium stearate.

### Tablets

The pharmaceutical composition of the present invention, in solid form, may be prepared in the form of tablets for example: non-coated tablets, coated tablets, or immediate release tablets. In general, the pharmaceutical composition is prepared by uniformly and intimately bringing the pharmaceutical composition of the present invention into association with one or more solid pharmaceutically acceptable excipients, and then, if necessary, shaping the product into the desired formulation.

For examples, conventional methods for preparing tablets are known. Such methods include dry methods such as direct compression and compression of granulation produced by compaction, or wet methods or other special procedures.

### Parenteral formulations

The pharmaceutical composition of the present invention may be prepared in unit dosage form for parenteral administrations including: intramuscular injections, intrathecal injections, intradermal injections, intraperitoneal injections, and subcutaneous injection.

In one embodiment of the present invention, the parenteral formulations may be prepared in the form of sterile solutions or suspensions, by employing conventional procedures well known in the art. For example, the composition may be filled into ampoule or glass vial; optionally with the addition of pharmaceutically acceptable excipients, and sterilized by the commonly used techniques. The composition may take such form as suspension, solution or emulsion, and may contain suspending, stabilizing and/or dispersing agents.

Furthermore, the pharmaceutical composition of the present invention may be in powder form for constitution with a suitable vehicle, e.g., sterile pyrogen-free water, before use.

### Sustained release formulations

Alternatively, the pharmaceutical composition of the present invention may be delivered using a sustained-release system to provide controlled release of calanolide to allow less frequent dosing or to improve the pharmacokinetic or toxicity profile of the active ingredient. Various types of sustained-release materials have been established and are well known by those skilled in the art. For examples, the composition of the present invention may be formulated as a sustained-release tablet or a double-layer tablet with one layer of immediate release and one layer of sustained-released of the said composition.

Alternatively, the pharmaceutical composition of the present invention may be delivered using a sustained-release system to provide controlled release of calanolide to allow less frequent dosing or to improve the pharmacokinetic or toxicity profile of the active ingredient. Various types of sustained-release materials have been established and are well known by those skilled in the art. For examples, the pharmaceutical composition of the present invention may be formulated as a sustained-release tablet or a double-layer tablet with one layer of immediate release and one layer of sustained-released of the said composition.

### Use of the compositions

In another embodiment, the present invention provides a process of increasing the oral or parenteral bioavailability of calanolide, comprising: by orally or parenterally administering to a mammal, especially a human, in need of such composition a therapeutically effective amount of a pharmaceutical composition comprising: a calanolide, one or more solubility enhancers, and one or more surfactants. The said process enhances the bioavailability of calanolide administered by improving solubility of calanolide.

In a preferred embodiment; the present invention provides a process of increasing the oral or parenteral bioavailability of calanolide comprising, by orally or parenterally administering to a subject in need of such composition a therapeutically effective amount of the said composition comprising: a calanolide in an amount of from 0.5% to about 80%, a solubility enhancer in an amount of about 0.5% to about 90%, and a surfactant in an amount of from about 0.1% to about 80%, by weight of the total composition.

In accordance with one aspect of the invention, there are provided pharmaceutical compositions for the use in the treatment and prevention of retroviral diseases such as human immunodeficiency, specifically HIV-1 and mycobacterial diseases especially tuberculosis infections in mammals, particularly in humans, wherein the said compositions have enhanced solubility and bioavailability.

As one skilled in the art will appreciate, the pharmaceutical compositions can also be used to will inhibit other retroviruses and may inhibit viruses, other than retroviruses. Examples of viruses that may be treated in accordance with the present invention include, but are not limited to, Type C and Type D retroviruses, HTLV-1, HTLV-2, HIV, FLV, SIV, MLV, BLV, BIV, equine infectious, anemia virus, avian sarcoma viruses, such as rous sarcoma virus (RSV), hepatitis type A, B, non-A, and non-B viruses, herpes viruses, cytomegaloviruses, influenza viruses, arboviruses, varicella viruses, measles, mumps and rubella viruses.

In one embodiment, the present invention provides the use of compositions according to the present invention for the manufacture of medicaments for the treatment and prevention of retroviral diseases such as human immunodeficiency, specifically HIV-1 and mycobacterial diseases especially tuberculosis infections in mammals, preferably humans, wherein the said compositions have enhanced solubility and bioavailability.

In yet another aspect, the present invention provides a process of enhancing the bioavailability of calanolide in a subject (e.g., a mammal, particularly a human) comprising the step of: administering, by oral or parenteral route, to a subject in need of such treatment a therapeutically effective amount of the pharmaceutical composition of the present invention.

### Dosage

In another embodiment of the invention, the dosage and the number of times of administration of the pharmaceutical composition to a patient may be varied and will depend on several factors such as: the age and body weight of the patient, sex, the condition of the patient, the mode of administration, and will be a matter to be determined by the attending physician.

In humans the daily dose can be expected to be from about 3 mg to about 50 mg of calanolide per kg body weight, conveniently administered, for example, in divided doses of up to four times a day or once daily.

A more preferred daily dose is from about 10 mg to about 30 mg of calanolide per kg body weight and the most preferred daily dose is from about 15 mg to about 25 mg of calanolide per kg body weight.

It should be appreciated that daily doses other than those described above may be administered to a subject, as determined by the attending physician.

### Dosage per unit form

In one embodiment of the present invention, the amount of calanolide contained in an oral unit dosage form may be generally varied from about 10 mg to about 1000 mg of calanolide, and more preferably from 20 to 200 mg, e.g.: 25, 50, 100, 125, 150, or 200 mg of calanolide. Most preferably, the oral unit dosage form contains about 100 mg to about 150 mg of calanolide.

In another embodiment of the present invention, the amount of calanolide contained in a parenteral unit dosage form may be generally varied from about 5 mg to about 500 mg of calanolide, and more preferably from 20 to 200 mg, e.g.: 25, 50, 100, 125, 150, or 200 mg of calanolide. Most preferably, the oral unit dosage form contains about 50 mg to about 100 mg of calanolide.

### Methods of administration

In accordance with yet another embodiment of the present invention, there are provided methods of administration of pharmaceutical compositions of the invention to a subject in need thereof, said methods comprising: administering, by oral or parenteral route, a therapeutically effective amount of a pharmaceutical composition of the present invention, wherein said composition has enhanced solubility and bioavailability.

It is preferred that the pharmaceutical compositions of the present invention are administered in softgel capsule, capsule, tablet, oral solution, injectable solution, or the like form. In a more preferred embodiment, the composition is in a form adopted for oral administration in oral unit dosage form. Capsules, e.g.: soft or hard gelatin capsules, which represent the preferred oral dosage forms, are especially suitable unit dosage forms for oral administration.

### Combination Therapy

In one embodiment, the pharmaceutical composition of the present invention further comprises at least one additional anti-HIV agent. In a preferred embodiment, the anti-HIV agent can be selected from protease inhibitors, non-nucleoside reverse transcriptase inhibitors, nucleoside reverse transcriptase inhibitors, nucleotide reverse transcriptase inhibitors, integrease inhibitors, entry and fusion inhibitors, maturation inhibitors, and combinations thereof.

Preferred combinations include a pharmaceutical composition of the present invention and one or more other compounds selected from etravirine, efavirenz, delavirdine mesylate, nevirapine, lamivudine, zidovudine, emtricitabine, tenofovir disoproxil fumarate, didanosine, abacavir sulfate, stavudine, tipranavir, ritonavir, indinavir sulfate, darunavir, saquinavir mesylate, atazanavir sulfate, lopinavir, nelfinavir mesylate, fosamprenavir calcium, maraviroc, enfuvirtide, raltegravir, and combinations thereof.

In one embodiment, the pharmaceutical composition of the present invention further comprises at least one additional anti-mycobacterial agent. The anti-mycobacterial agent is anti-TB agent selected from isoniazid, rifampin, rifabutin, rifapentine, ethambutol, cyclic peptides, ethionamide, fluoroquinolones, pyrazinamide, PAS (*p*-aminosalicylic acid), aminoglycosides, cycloserine, thioamides, linezolid, levofloxacin, ciprofloxacin, moxi-or gatifloxacin, amikacin, kanamycin, streptomycin, capreomycin, viomycin, enviomycin, clarithromycin, thioacetazone, thioridazine, arginine, vitamin D and combinations thereof.

In yet another aspect, the present invention provides a method of use of the pharmaceutical compositions of the present invention for treating and preventing retroviral diseases, in particular HIV infection in mammals, comprising administering to a subject in need thereof a therapeutically effective amount of pharmaceutical composition of the present invention in combination with at least one additional anti-HIV agent selected from protease inhibitors, non-nucleoside reverse transcriptase inhibitors, nucleoside reverse transcriptase inhibitors, nucleotide reverse transcriptase inhibitors, integrase inhibitors, entry and fusion inhibitors, maturation inhibitors, and combinations thereof.

Further in yet another aspect, the present invention provides a method of use of the pharmaceutical compositions of the present invention for treating and preventing mycobacterial diseases especially tuberculosis infections in mammals, comprising administering to a subject in need thereof a therapeutically effective amount of pharmaceutical composition of the present invention in combination with at least one additional anti-TB agent selected from isoniazid, rifampin, rifabutin, rifapentine, ethambutol, cyclic peptides, ethionamide, fluoroquinolones, pyrazinamide, PAS (*p*-aminosalicylic acid), aminoglycosides, cycloserine, thioamides, linezolid, levofloxacin, ciprofloxacin, moxi-or gatifloxacin, amikacin, kanamycin, streptomycin, capreomycin, viomycin, enviomycin, clarithromycin, thioacetazone, thioridazine, arginine, vitamin D and combinations thereof.

Such combinations are selected based on the condition to be treated, cross-reactivity of ingredients and pharmaco-properties of the combination. Such other drugs may be administered, by a route and in an amount commonly used, contemporaneously or sequentially with a composition of the present invention.

The combination therapy may provide "synergy" and "synergistic effect", i.e. the effect achieved when the active ingredients used together is greater than the sum of the effects that results from using the compounds separately. A synergistic effect may be attained when the active ingredients are: (1) co-formulated and administered or delivered simultaneously in a combined formulation; (2) delivered by alternation or in parallel as separate formulations; or (3) by some other regimen. When delivered in alternation therapy, a synergistic effect may be attained when the compounds are administered or delivered sequentially, e.g., in separate tablets, pills or capsules, or by different injections in separate syringes. In general, during alternation therapy, an effective dosage of each active ingredient is administered sequentially, i.e. serially, whereas in combination therapy, effective dosages of two or more active ingredients are administered together.

It is also possible to combine any pharmaceutical composition of the present invention with one or more other active ingredients in a unit dosage form for simultaneous or sequential administration to a patient. The combination therapy may be administered as a simultaneous or sequential regimen. When administered sequentially, the combination may be administered in two or more administrations per day.

These combination treatments are expected to be particularly effective for the treatment or control of HIV infections and mycobacterial diseases in mammals, particularly, humans. The combination therapy may make it possible to achieve therapeutic control using a reduced amount of one or both active ingredients and/or to achieve better control than would be expected based on the control that is achieved when either of the compositions is used alone.

The pharmaceutical compositions of the present invention will be better understood in connection with the following examples, which are intended as an illustration of and not a limitation upon the scope of the invention.

It should be recognized that various modifications and changes may be made to the invention by those skilled in the art which also fall within the scope of the invention as defined by the appended claims.

The examples are described with particular reference to calanolide A. However, equivalent compositions may be obtained by employing any other appropriate calanolides.

Representative formulations of pharmaceutical compositions of the invention are exemplified in Examples 1 - 8. Results of solubility tests are found in Example 9. Results of oral bioavailability test in rats are found in Example 10, and results of parenteral bioavailability test in rats are found in Example 11.

### Control Composition (Comparative Example A)

For comparative purposes, a control composition according to the following formula was prepared:

| **Ingredients** | **mg / unit dose** | **% w/w** |
|---|---|---|
| Calanolide-A | 100.0 | 12.12 |
| Sesame oil | 725.0 | 87.88 |
| Total | 825.0 | |

7.25 kg of sesame oil was heated to about 50°C - 60°C, and 1.0 kg of calanolide A is added and dissolved in sesame oil. The liquid was then allowed to cool to room temperature. Each 825 mg of the liquid contained 100 mg of calanolide A. 825 mg of the mixture was then filled into each soft gelatin capsule containing 100 mg of calanolide A suitable for oral administration.

Representative formulations of pharmaceutical compositions made according to the invention are set forth below:

### EXAMPLE 1

### Formulation for Solution of Calanolide A

The following formulation provides a solution of calanolide A suitable as a softgel fill and has an enhanced water solubility and bioavailability as shown in Examples 9 and 10 hereinafter.

| **Ingredients** | **mg / soft capsule** | **% w/w** |
|---|---|---|
| (+)-Calanolide A | 100.0 | 12.12 |
| Medium chain triglyceride oil (Miglyol 810) | 560.0 | 67.88 |
| PEG-40 hydrogenated castor oil (Cremophor RH 40) | 165.0 | 20.00 |

5.6 kg of medium-chain triglyceride oil was heated to about 50°C - 60°C, and 1.0 kg of calanolide A was added and dissolved in the oil. 1.65 kg of PEG-40 hydrogenated castor oil (Cremophor RH 40) was added to the mixture which was allowed to cool to room temperature. 825 mg of the mixture was then filled into each soft gelatin capsule containing 100 mg of calanolide A suitable for oral administration.

### EXAMPLE 2

### Formulation for Solution of Calanolide A

The following formulation provides a solution of calanolide A suitable as a softgel fill and has an enhanced water solubility and bioavailability as shown in Examples 9 and 10 hereinafter.

| **Ingredient** | **mg / soft capsule** | **% w/w** |
|---|---|---|
| (+)-Calanolide A | 100.0 | 12.50 |
| Medium chain triglyceride oil (Miglyol 810) | 100.0 | 12.50 |
| Polyethylene glycol 400 | 200.0 | 25.00 |
| Polysorbate 80 (TWEEN 80) | 380.0 | 47.50 |
| Sorbitan monolaurate (SPAN 20) | 20.0 | 2.50 |
| Total | 800.0 | |

1.0 kg of medium-chain triglyceride oil and 2.0 kg of polyethylene glycol 200 were heated to about 50°C - 60°C, and 1.0 kg of calanolide A was added and dissolved in the mixture. 3.8 kg of Polysorbate 80 and 0.2 kg of sorbitan monolaurate were then added to the mixture which was allowed to cool to room temperature. 800 mg of the mixture was then filled into each soft gelatin capsule containing 100 mg of calanolide A suitable for oral administration.

### EXAMPLE 3

### Formulation for Solution of Calanolide A

The following formulation provides a solution of calanolide A suitable to be filled aseptically for parenteral (intramuscular or subcutaneous) administration and has an enhanced water solubility and bioavailability as shown in Examples 9 and 11 hereinafter.

| **Ingredients** | **mg / vial** | **% w/w** |
|---|---|---|
| (+)-Calanolide A | 100.0 | 12.50 |
| N-methylpyrrolidone (Pharmasolve, ISP) | 632.0 | 79.00 |
| Polysorbate 20 (TWEEN 20) | 40.0 | 5.00 |
| PEG-40 hydrogenated castor oil (Cremophor RH 40) | 28.0 | 3.50 |
| Total | 800.0 | |

1.0 kg of calanolide A was dissolved in 6.32 kg of N-methylpyrrolidone at 20°C - 30°C. 0.4 kg of Polysorbate 20 and 0.28 kg of PEG-40 hydrogenated castor oil were added to the mixture with stirring until a homogenous solution was formed. 800 mg of the mixture was aseptically filled into each glass vial for injection.

Each vial contained 100 mg of calanolide A suitable for intramuscular or subcutaneous administration.

### EXAMPLE 4

### Formulation for Solution of Calanolide A

The following formulation provides a solution of calanolide A suitable as a liquid fill into hard capsule and has enhanced water solubility as shown in Example 9 hereinafter.

| **Ingredients** | **mg / capsule** | **% w/w** |
|---|---|---|
| (+)-Calanolide A | 50.0 | 11.10 |
| Polyethylene glycol 200 | 226.8 | 50.40 |
| Polysorbate 80 (TWEEN 80) | 173.2 | 38.49 |
| Total | 450.0 | |

2.268 kg of polyethylene glycol 200 was heated to about 50°C - 60°C, and 0.5 kg of calanolide A was added and dissolved in the liquid. 1.732 kg of Polysorbate 80 was added to the mixture and the mixture was allowed to cool to room temperature. 450 mg of the mixture was then filled into each hard shell capsule containing 50 mg of calanolide A suitable for oral administration.

### EXAMPLE 5

### Formulation for Solution of Calanolide A

The following formulation provides a solution of calanolide A suitable to be filled aseptically for parenteral (intramuscular or subcutaneous) administration and has enhanced water solubility as shown in Example 9 hereinafter.

| **Ingredients** | **mg / vial** | **% w/w** |
|---|---|---|
| (+)-Calanolide A | 100.0 | 10.00 |
| 2-Pyrrolidone (2-Pyrol®) | 500.0 | 50.00 |
| PEG-40 hydrogenated castor oil (Cremophor RH 40) | 400.0 | 40.00 |
| Total | 1000.0 | |

10.0 kg of calanolide A was dissolved in 50.0 kg of 2-pyrrolidone at 20°C - 30°C. 40.0 kg of PEG-40 hydrogenated castor oil was added to the mixture with stirring until a homogenous solution was formed. 1000 mg of the mixture was aseptically filled into each glass vial for injection. Each vial contained 100 mg of calanolide A suitable for intramuscular or subcutaneous administration.

### EXAMPLE 6

### Formulation for Solution of Calanolide A

The following formulation provides a solution of calanolide A suitable as a softgel fill.

| **Ingredients** | **mg / capsule** | **% w/w** |
|---|---|---|
| (+)-Calanolide A | 25.0 | 5.0 |
| Sesame oil | 350.0 | 70.0 |
| Apricot kernel oil PEG-6-ester (Labrafil M 1944 CS) | 125.0 | 25.0 |
| Total | 500.0 | |

0.35 kg of sesame oil was heated to about 50°C - 60°C, and 0.025 kg of calanolide A was added and dissolved in the sesame oil. 0.125 kg of apricot kernel oil PEG-6-ester was added to the mixture which was allowed to cool to room temperature. The mixture was then filled into soft gelatin capsules. Each soft gelatin capsule contained 25 mg of calanolide A suitable for oral administration.

### EXAMPLE 7

### Formulation for Calanolide A Tablet

The following formulation provides a powder mixture of calanolide A suitable to be compressed into tablets.

| **Ingredients** | **mg / tablet** | **% w/w** |
|---|---|---|
| (+)-Calanolide A | 200.0 | 25.0 |
| Beta-cyclodextrin | 412.5 | 51.6 |
| Polysorbate 80 (TWEEN 80) | 15.0 | 1.9 |

| **Excipients** | | |
|---|---|---|
| Microcrystalline cellulose | 120.0 | 15.0 |
| Sodium starch glycolate | 15.0 | 1.9 |
| Polyvinylpyrrolidone K-30 (Povidone K-30) | 22.5 | 2.8 |
| Silicon dioxide (AEROSIL® 200) | 7.5 | 0.9 |
| Magnesium stearate | 7.5 | 0.9 |
| Total | 800.0 | |

2.0 kg of calanolide A was mixed with 4.125 kg of beta-cyclodextrin, and the powder was milled in a ball mill for 20 hours. 0.15 kg of Polysorbate 80 was added to the mixture and mixed until a homogenous powder was formed. 1.2 kg of microcrystalline cellulose, 0.15 kg of sodium starch glycolate, 0.225 kg of polyvinylpyrrolidone K-30, and 0.075 kg of silicone dioxide were added to the powder and mixed for 15 minutes. 0.075 kg of magnesium stearate was then added and mixed for 5 minutes. The powder was compressed into tablets on a tableting machine using 19 mm punches. Each 800 mg of tablet contained 200 mg of calanolide A suitable for oral administration.

### EXAMPLE 8

### Formulation for Calanolide A Hard Capsule

The following formulation provides a powder of calanolide A suitable to be filled into hard capsules.

| **Ingredients** | **mg / capsule** | **% w/w** |
|---|---|---|
| (+)-Calanolide A | 275.0 | 55.0 |
| Corn oil | 50.0 | 10.0 |
| Polyethylene glycol 4000 | 155.0 | 31.0 |
| PEG-35 castor oil (Cremophor ® EL) | 20.0 | 4.0 |
| Total | 500.0 | |

1.55 kg of polyethylene glycol 4,000 and 0.5 kg of corn oil were heated to about 50°C - 60°C, and 2.75 kg of calanolide A was added and dissolved in the hot liquid. 0.2 kg of PEG-35 castor oil was added to the mixture and the mixture was allowed to cool to room temperature. 500 mg of the resulting powder mixture was then filled into each hard capsule containing 275 mg of calanolide A suitable for oral administration.

### EXAMPLE 9

### Solubility Tests

### Test Method

These tests were performed to compare the solubility of calanolide A of the representative formulations of pharmaceutical compositions of the invention and that of the control composition as set forth in Comparative Example A.

The solubility tests were carried out in accordance with *U S Pharmacopoeia XXII, Dissolution Method II,* (Paddle method).

Test samples, each containing 100 mg of calanolide A, were added to 900 ml of purified water. The mixture was stirred at 50 rpm for 30 minutes and maintained at 37°C. The aliquots were then collected and filtered through a 0.45 µm Millipore filter. The amount of dissolved calanolide was assayed by a high performance liquid chromatography (HPLC) method for calanolide as follows:

| HPLC Conditions | | | | |
|---|---|---|---|---|
| Column | Hypersil BDS C₁₈, 250 x 4.6 mm (length x I.D), 5 µm particle size. | | | |
| Detection | UV at 254 nm | | | |
| Flow rate | 1.0 mL/min | | | |
| Injection volume | 20 µL | | | |
| Run time | 12 minutes | | | |
| Mobile phase | Solvent A: Methanol: Water (90:10), Solvent B: Methanol | | | |
| Analysis mode | Gradient | | | |

| | Time (min) | Solvent A (%) | Solvent B (%) | Elution |
|---|---|---|---|---|
| | 0 - 1 | 100 | 0 | Isocratic |
| | 1 - 3 | 5 | 95 | Linear gradient |
| | 3 - 10 | 5 | 95 | Isocratic |
| | 10 - 12 | 100 | 0 | Linear gradient |

The solubility values of formulations of Examples 1 - 6 and formulation of the control composition (Comparative Example A) are set forth in Table 1. (The dissolved Calanolide A is expressed in milligrams/milliliter).

**TABLE 1: Solubility Values of Calanolide A**

| **Samples** | **Solubility (mg/ml)** |
|---|---|
| Comparative Example A | 0.0016 ± 0.0002 |
| Composition 1 (as per Example 1) | 0.1296 ± 0.0067 |
| Composition 2 (as per Example 2) | 0.1115 ± 0.0100 |
| Composition 3 (as per Example 3) | 0.1129 ± 0.0173 |
| Composition 4 (as per Example 4) | 0.1235 ± 0.0095 |
| Composition 5 (as per Example 5) | 0.1296 ± 0.0068 |

As shown in Table 1, it is apparent that the representative formulations of pharmaceutical compositions prepared in accordance with the present invention have a remarkably enhanced solubility.

The solubility of the representative formulations of pharmaceutical compositions of the present invention, e.g. Example 1 - 5, is at least 60 times more than that of the control composition as set forth in Comparative Example A

### EXAMPLE 10

### Oral Bioavailability Test in Rats

Oral bioavailability tests on the representative formulations of pharmaceutical compositions according to the present invention, as prepared in Example 1 and Example 2, were compared to that of the control composition as set forth in Comparative Example A.

In this experiment, the representative formulations of pharmaceutical composition of the present invention of Examples 1 and 2, and the control composition of Comparative Example A were orally administered, by using oral gavage, to a group of male Sprague-Dawley rats (n=3) at a dose of 100 mg of calanolide A per kg body weight. Water was available *ad libitum.* Each rat, in the weight range of 300-400 g, was fasted overnight prior to dosing.

Serial blood samples of 0.25 ml were obtained from the tail vein at 0.5, 1, 2, 5, 6, and 24 hours after dosing and then centrifuged to provide plasma samples. The plasma levels of Calanolide A were determined by the HPLC method as described in Example 9.

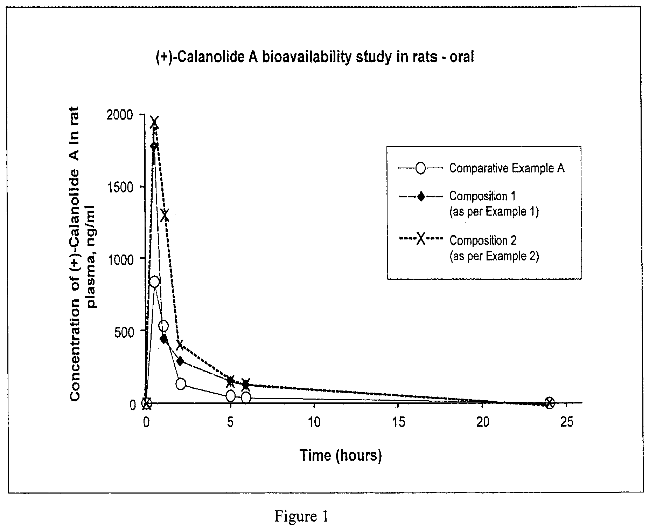

The concentrations of Calanolide A in the blood of the test rats were plotted as a function of time. Fig. 1 shows the plasma/serum concentration-time curves for Calanolide A in rats following oral administration of pharmaceutical compositions according to the invention (Examples 1 and 2) in comparison with that of the control composition (Comparative Example A).

The AUC₀₋₂₄ (the Area Under the Plasma Concentration-Time Curve) values were calculated from the curve using the trapezoidal rule and set forth in Table 2.

**TABLE 2: AUC₀₋₂₄ values of calanolide A for rats after oral administration of Calanolide A.**

| **Blood calanolide A level (ng/ml ± S.D) (n = 3)** | | | | | | | | **Parameters** | |
|---|---|---|---|---|---|---|---|---|---|
| Time after administration (Hour) | 0 | 0.5 | 1 | 2 | 5 | 6 | 24 | Cmax ngml⁻¹ | AUC (0 - 24 hr) ngml⁻¹.h |
| Control composition (Comparative Example A) | 0 | 840.03 ± 160.75 | 537.68 ± 95.15 | 135.4 ± 7.75 | 51.55 ± 12.37 | 41.3 ± 1.70 | 0 | 840.03 ± 160.75 | 1791.46 |
| Composition 1 (as per Example 1) | 0 | 1952.88 ± 231.25 | 1305.59 ± 211.55 | 401.95 ± 36.56 | 151.75 ± 20.15 | 127.15 ± 13.22 | 0 | 1952.88 ± 231.25 | 4734.57 |
| Composition 2 (as per Example 2) | 0 | 1779.34 ± 542.72 | 453.5 ± 227.55 | 289.45 ± 64.23 | 159 ± 16.26 | 126.11 ± 24.59 | 0 | 1779.34 ± 542.72 | 3776.71 |

The average AUC₀₋₂₄ values of the representative formulations of pharmaceutical compositions prepared in Example 1 and Example 2 according to the present invention are substantially higher than those of the control composition prepared in Comparative Example A.

From Table 2 it is apparent that the representative formulations of pharmaceutical compositions according to the invention yield higher degree of oral bioavailability for calanolide A than that of control composition, which was determined in the same manner and at the same dosage.

The oral bioavailabilities, expressed in terms of the AUC₀₋₂₄ values, of the representative formulations of pharmaceutical compositions according to the invention are, surprisingly, more than 200% higher than that of the control composition.

### EXAMPLE 11

### Parenteral Bioavailability Test in Rats

Parenteral bioavailability tests on the representative formulation of pharmaceutical composition according to the present invention as prepared in Example 3 was compared to that of the control composition prepared in Comparative Example A.

In this experiment, the representative formulation of pharmaceutical composition of the present invention of Example 3 and the control composition of Comparative Example A were administered by intramuscular injection, to a group of male Sprague-Dawley rats (n=3) at a dose of 15 mg of calanolide A per kg body weight. Water was available *ad libitum.* Each rat, in the weight range of 300-400 g, was fasted overnight prior to dosing.

Serial blood samples of 0.25 ml were obtained from the tail vein at 0.5, 1.5, 2, 5, 7 and 24 hours after dosing and then centrifuged to provide plasma samples. The plasma levels of calanolide A were determined by the HPLC method as described in Example 9.

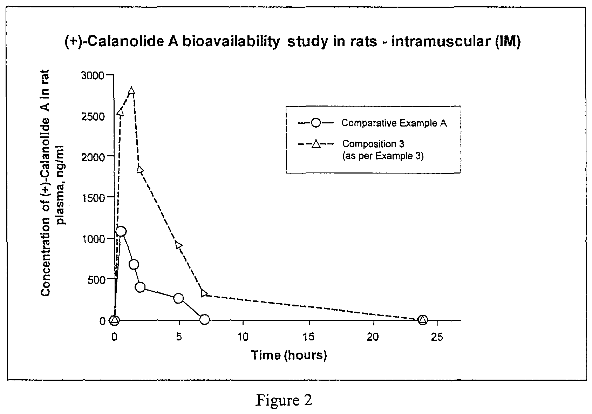

The concentrations of calanolide A in the blood of the test rats were plotted as a function of time. Fig. 2 shows the plasma/serum concentration-time curves for Calanolide A in rats following intramuscular administration of pharmaceutical composition according to the invention (Example 3) in comparison with that of the control composition (Comparative Example A).

The AUC₀₋₂₄ (the Area Under the Plasma Concentration-Time Curve) values were calculated from the curve using the trapezoidal rule and set forth in Table 3.

**TABLE 3: AUC₀₋₂₄ values of Calanolide A for rats after intramuscular administration of Calanolide A.**

| **Blood Calanolide A level (ng/ml ± S.D) (n = 3)** | | | | | | | | **Parameters** | |
|---|---|---|---|---|---|---|---|---|---|
| Time after administration (Hours) | 0 | 0.5 | 1.5 | 2 | 5 | 7 | 24 | Cmax ngml⁻¹ | AUC (0 - 24 hr) ngml⁻¹.h |
| Control composition (Comparative Example A) | 0 | 1099.1 ± 8.66 | 679.3 ± 16.69 | 397.33 ± 57.45 | 254.04 + 39.25 | 0 | 0 | 1099.1 ± 8.66 | 2664.84 |
| Composition 3 (as per Example 3) | 0 | 2556.48 ± 229.03 | 2819.84 ± 1031.24 | 1822.29 ± 412.54 | 899.06 ± 66.57 | 290.12 ± 17.93 | 0 | 2556.48 ± 229.03 | 12225.03 |

The average AUC₀₋₂₄ value of the representative formulation of pharmaceutical composition prepared in Example 3 according to the present invention is substantially higher than that of the control composition prepared in Comparative Example A.

From Table 3, it is apparent the representative formulation of pharmaceutical composition according to the invention yields higher degree of parenteral (intramuscular) bioavailability for calanolide A than that of the control composition, which was determined in the same manner and at the same dosage.

The parenteral (intramuscular) bioavailability, expressed in terms of the AUC₀₋₂₄ value, of the representative formulation of pharmaceutical composition according to the invention is, surprisingly, more than 400% higher than that of the control composition.

Hence pharmaceutical compositions of the present invention comprising calanolide provide an increase in oral or parenteral bioavailability in AUC₀₋₂₄ of calanolide at least 20 percent greater than that of the same calanolide in a control composition (Comparative Example A).
Pharmaceutical compositions of the present invention comprising calanolide also provide an increase in oral or parenteral bioavailability in Cₘₐₓ of calanolide at least 30 percent greater than that of the same calanolide in a control composition (Comparative Example A).

### REFERENCES

1) Terri Creagh et al., "Safety and Pharmacokinetics of Single Doses of (+)-Calanolide A, a Novel, Naturally Occurring Nonnucleoside Reverse Transcriptase Inhibitor, in Healthy, Human Immunodeficiency Virus-Negative Human Subjects", Antimicrobial Agents and Chemotherapy, vol. 45, No. 5, pp. 1379-1386 (May 2001).
2) David A. Eiznhamer et al., "Safety and Pharmacokinetic Profile of Multiple Escalating Doses of (+)-Calanolide A, a Naturally Occurring Nonnucleoside Reverse Transcriptase Inhibitor, in Healthy HIV-Negative Volunteers", HIV Clin Trials, vol. 3, No. 6, pp. 435-450 (2002).

## Claims

1. A pharmaceutical composition of calanolide, for oral or parenteral administration, comprising:
a) a calanolide or mixture of calanolides,
b) one or more solubility enhancers selected from the group consisting of sesame oil, olive oil, glyceryl tricaprylate/caprate, glyceryl tricaprylate/caprate/linoleate, glyceryl monocaprate, glyceryl monocaprylate, glyceryl monostearate, glyceryl monooleate, glyceryl dicaprylate, glyceryl dilaurate, isopropyl myristate, isopropyl oleate, 2-pyrrolidone, N-methyl-2-pyrrolidone, beta-cyclodextrin, hydroxypropyl beta-cyclodextrin, polyethylene glycol 200, polyethylene glycol 300, polyethylene glycol 400 and mixtures thereof, and
c) one or more surfactants selected from the group consisting of PEG-20 sorbitan monolaurate, PEG-20 sorbitan monopalmitate, PEG-20 sorbitan monostearate, PEG-20 sorbitan monooleate, PEG-40 hydrogenated castor oil, PEG-35 castor oil, PEG-6 apricot kernel oil and mixtures thereof.

2. The pharmaceutical composition according to claim 1, wherein said composition is in the form of liquid, semi-solid, or solid.

3. The pharmaceutical composition according to claim 1 or 2, wherein:
a) the calanolide or mixture of calanolides is present in an amount of from 0.5% to 80% by weight of the total composition;
b) the solubility enhancer is present in an amount of from 0.5% to 90% by weight of the total composition; and
c) the surfactant is present in an amount of from 0.1 % to 80% by weight of the final composition;
or
a) the calanolide or mixture of calanolides is present in an amount of from 5% to 50% by weight of the total composition;
b) the solubility enhancer is present in an amount of from 10% to 60% by weight of the total composition; and
c) the surfactant is present in an amount of from 5% to 50% by weight of the final composition;
or
a) the calanolide or mixture of calanolides is present in an amount of from 10% to 35% by weight of the total composition;
b) the solubility enhancer is present in an amount of from 25% to 50% by weight of the total composition; and
c) the surfactant is present in an amount of from 20% to 40% by weight of the final composition.

4. The pharmaceutical composition according to any of the preceding claims, wherein the calanolide is selected from the group consisting of calanolides, their pharmaceutically acceptable salts, esters, amides, isomers, and mixtures thereof.

5. The pharmaceutical composition according to any of the preceding claims, wherein the calanolide particles present in the composition are in the form of crystalline, semi-crystalline, amorphous, semi-amorphous, liquid, dispersed solid, or combination(s) of form thereof.

6. The pharmaceutical composition according to any of the preceding claims, further comprising at least one pharmaceutically acceptable excipient.

7. The pharmaceutical composition according to any of the preceding claims, wherein said composition is present in a unit dosage form for oral or parenteral administration.

8. The pharmaceutical composition according to any of the preceding claims, further comprising at least one additional agent wherein the additional agent is anti-HIV agent, anti-mycobacterial agent, or a combination thereof.

9. A process of preparing the pharmaceutical composition according to any of the preceding claims comprising the steps of:
i) mixing a calanolide or mixture of calanolides with one or more solubility enhancers as defined in claim 1, at temperature between 20°C to 65°C;
ii) mixing the resulting mixture obtained in step (i) with one or more surfactants as defined in claim 1 to form a homogenous mixture; and
wherein the mixing process in step (i) can further comprise an additional step of grinding the powder mixture in a dispersion mill for 1 to 40 hours,
or
i) mixing a calanolide or mixture of calanolides in an amount of from 0.5% to 80% by weight of the total composition with one or more solubility enhancers as defined in claim 1 in an amount of from 0.5% to 90% by weight of the total composition, at temperature between 20°C to 65°C;
ii) mixing the resulting mixture obtained in step (i) with one or more surfactants as defined in claim 1 in an amount of from 0.1 % to 80% to form a homogenous mixture; and
wherein the mixing process in step (i) can further comprise an additional step of grinding the powder mixture in a dispersion mill for 1 to 40 hours,
or
i) mixing a calanolide or mixture of calanolides in an amount of from 5% to 50% by weight of the total composition with one or more solubility enhancers as defined in claim 1 in an amount of from 10% to 60% by weight of the total composition, at temperature between 20°C to 65°C;
ii) mixing the resulting mixture obtained in step (i) with one or more surfactants as defined in claim 1 in an amount of from 5 % to 50% to form a homogenous mixture; and
wherein the mixing process in step (i) can further comprise an additional step of grinding the powder mixture in a dispersion mill for 1 to 40 hours,
or
i) mixing a calanolide or mixture of calanolides in an amount of from 10% to 35% by weight of the total composition with one or more solubility enhancers as defined in claim 1 in an amount of from 25% to 50% by weight of the total composition, at temperature between 20°C to 65°C;
ii) mixing the resulting mixture obtained in step (i) with one or more surfactants as defined in claim 1 in an amount of from 20 % to 40% to form a homogenous mixture; and
wherein the mixing process in step (i) can further comprise an additional step of grinding the powder mixture in a dispersion mill for 1 to 40 hours.

10. The pharmaceutical composition according to any of claims 1-8 for use in the treatment and prevention of retroviral diseases in mammals.

11. The pharmaceutical composition according to any of claims 1-8 for use in the treatment and prevention of mycobacterial diseases in mammals.

12. The pharmaceutical composition of any of claims 1-8 for the use according to claim 10 or 11, wherein the mammal is a human.

## Patentansprüche

1. Pharmazeutische Zusammensetzung von Calanolid zur oralen oder parenteralen Verabreichung, umfassend:
a) ein Calanolid oder Mischung von Calanoliden,
b) ein oder mehrere Löslichkeitsverstärker ausgewählt aus der Gruppe bestehend aus Sesamöl, Olivenöl, Glyceryltricaprylat/caprat, Glyceryltricaprylat/caprat/linoleat, Glycerylmonocaprat, Glycerylmonocaprylat, Glycerylmonostearat, Glycerylmonooleat, Glyceryldicaprylat, Glyceryldilaurat, Isopropylmyristat, Isopropyloleat, 2-Pyrrolidon, N-Methyl-2-pyrrolidon, beta-Cyclodextrin, Hydroxypropyl-beta-Cyclodextrin, Polyethylenglycol 200, Polyethylenglycol 300, Polyethylenglycol 400 und Mischungen davon, und
c) ein oder mehrere Tenside ausgewählt aus der Gruppe bestehend aus PEG-20 Sorbitanmonolaurat, PEG-20 Sorbitanmonopalmitat, PEG-20 Sorbitanmonostearat, PEG-20 Sorbitanmonooleat, PEG-40 hydriertes Rizinusöl, PEG-35 Rizinusöl, PEG-6 Aprikosenkernöl und Mischungen davon.

2. Pharmazeutische Zusammensetzung nach Anspruch 1, wobei die Zusammensetzung in der Form einer Flüssigkeit, halbfest oder fest ist.

3. Pharmazeutische Zusammensetzung nach Anspruch 1 oder 2, wobei:
a) das Calanolid oder die Mischung von Calanoliden in einer Menge von 0,5 Gew.-% bis 80 Gew.-% der Gesamtzusammensetzung vorhanden ist;
b) der Löslichkeitsverstärker in einer Menge von 0,5 Gew.-% bis 90 Gew.-% der Gesamtzusammensetzung vorhanden ist; und
c) das Tensid in einer Menge von 0,1 Gew.-% bis 80 Gew.-% der Endzusammensetzung vorhanden ist;
oder
a) das Calanolid oder die Mischung von Calanoliden in einer Menge von 5 Gew.-% bis 50 Gew.-% der Gesamtzusammensetzung vorhanden ist;
b) der Löslichkeitsverstärker in einer Menge von 10 Gew.-% bis 60 Gew.-% der Gesamtzusammensetzung vorhanden ist; und
c) das Tensid in einer Menge von 5 Gew.-% bis 50 Gew.-% der Endzusammensetzung vorhanden ist;
oder
a) das Calanolid oder die Mischung von Calanoliden in einer Menge von 10 Gew.-% bis 35 Gew.-% der Gesamtzusammensetzung vorhanden ist;
b) der Löslichkeitsverstärker in einer Menge von 25 Gew.-% bis 50 Gew.-% der Gesamtzusammensetzung vorhanden ist; und
c) das Tensid in einer Menge von 20 Gew.-% bis 40 Gew.-% der Endzusammensetzung vorhanden ist.

4. Pharmazeutische Zusammensetzung nach einem der vorhergehenden Ansprüche, wobei das Calanolid ausgewählt ist aus der Gruppe bestehend aus Calanoliden, ihren pharmazeutisch verträglichen Salzen, Estern, Amiden, Isomeren und Mischungen davon.

5. Pharmazeutische Zusammensetzung nach einem der vorhergehenden Ansprüche, wobei die Calanolidpartikel, die in der Zusammensetzung vorhanden sind, in kristalliner, halbkristalliner, amorpher, halbamorpher, flüssiger, disperser fester Form oder Kombination(en) der Formen davon sind.

6. Pharmazeutische Zusammensetzung nach einem der vorhergehenden Ansprüche, ferner umfassend zumindest einen pharmazeutisch verträglichen Exzipient.

7. Pharmazeutische Zusammensetzung nach einem der vorhergehenden Ansprüche, wobei die Zusammensetzung in einer Einheitsdosierungsform zur oralen oder parenteralen Verabreichung vorhanden ist.

8. Pharmazeutische Zusammensetzung nach einem der vorhergehenden Ansprüche, ferner umfassend zumindest ein zusätzliches Mittel, wobei das zusätzliche Mittel ein Anti-HIV-Mittel, ein gegen Mykobakterien wirkendes Mittel oder eine Kombination davon ist.

9. Verfahren zur Herstellung einer pharmazeutischen Zusammensetzung nach einem der vorhergehenden Ansprüche, umfassend die folgenden Schritte:
i) Mischen eines Calanolids oder einer Mischung von Calanoliden mit einem oder mehreren Löslichkeitsverstärkern nach Anspruch 1, bei einer Temperatur zwischen 20 °C bis 65 °C;
ii) Mischen der resultierenden Mischung, die in Schritt (i) erhalten wurde, mit einem oder mehreren Tensiden nach Anspruch 1, um eine homogene Mischung zu bilden; und
wobei das Mischverfahren in Schritt (i) ferner einen zusätzlichen Schritt des Zerkleinerns der Pulvermischung in einer Dispersionsmühle für 1 bis 40 Stunden umfassen kann
oder
i) Mischen eines Calanolids oder einer Mischung von Calanoliden in einer Menge von 0,5 Gew.-% bis 80 Gew.-% der Gesamtzusammensetzung mit einem oder mehreren Löslichkeitsverstärkern nach Anspruch 1 in einer Menge von 0,5 Gew.-% bis 90 Gew.-% der Gesamtzusammensetzung, bei einer Temperatur von zwischen 20 °C bis 65 °C;
ii) Mischen der resultierenden Mischung, die in Schritt (i) erhalten wurde, mit einem oder mehreren Tensiden nach Anspruch 1 in einer Menge von 0,1 Gew.-% bis 80 Gew.-%, um eine homogene Mischung zu bilden; und
wobei das Mischverfahren in Schritt (i) ferner einen zusätzlichen Schritt des Zerkleinerns der Pulvermischung in einer Dispersionsmühle für 1 bis 40 Stunden umfassen kann
oder
i) Mischen eines Calanolids oder einer Mischung von Calanoliden in einer Menge von 5 Gew.-% bis 50 Gew.-% der Gesamtzusammensetzung mit einem oder mehreren Löslichkeitsverstärkern nach Anspruch 1 in einer Menge von 10 Gew.-% bis 60 Gew.-% der Gesamtzusammensetzung, bei einer Temperatur von zwischen 20 °C bis 65 °C;
ii) Mischen der resultierenden Mischung, die in Schritt (i) erhalten wurde, mit einem oder mehreren Tensiden nach Anspruch 1 in einer Menge von 5 Gew.-% bis 50 Gew.-%, um eine homogene Mischung zu bilden; und
wobei das Mischverfahren in Schritt (i) ferner einen zusätzlichen Schritt des Zerkleinerns der Pulvermischung in einer Dispersionsmühle für 1 bis 40 Stunden umfassen kann
oder
i) Mischen eines Calanolids oder einer Mischung von Calanoliden in einer Menge von 10 Gew.-% bis 35 Gew.-% der Gesamtzusammensetzung mit einem oder mehreren Löslichkeitsverstärkern nach Anspruch 1 in einer Menge von 25 Gew.-% bis 50 Gew.-% der Gesamtzusammensetzung, bei einer Temperatur von zwischen 20 °C bis 65 °C;
ii) Mischen der resultierenden Mischung, die in Schritt (i) erhalten wurde, mit einem oder mehreren Tensiden nach Anspruch 1 in einer Menge von 20 Gew.-% bis 40 Gew.-%, um eine homogene Mischung zu bilden; und
wobei das Mischverfahren in Schritt (i) ferner einen zusätzlichen Schritt des Zerkleinerns der Pulvermischung in einer Dispersionsmühle für 1 bis 40 Stunden umfassen kann.

10. Pharmazeutische Zusammensetzung nach einem der Ansprüche 1 - 8 zur Verwendung in der Behandlung und Vorbeugung von retroviralen Erkrankungen bei Säugetieren.

11. Pharmazeutische Zusammensetzung nach einem der Ansprüche 1 - 8 zur Verwendung in der Behandlung und Vorbeugung von mykobakteriellen Erkrankungen bei Säugetieren.

12. Pharmazeutische Zusammensetzung nach einem der Ansprüche 1 - 8 zur Verwendung nach Anspruch 10 oder 11, wobei das Säugetier ein Mensch ist.

## Revendications

1. Composition pharmaceutique de calanolide, pour une administration par voie orale ou parentérale, comprenant :
a) un calanolide ou un mélange de calanolides ;
b) un ou plusieurs agent(s) favorisant la solubilité sélectionné(s) parmi le groupe qui est constitué par l'huile de sésame, l'huile d'olive, le tricaprylate/caprate de glycéryle, le tricaprylate/caprate/linoléate de glycéryle, le monocaprate de glycéryle, le monocaprylate de glycéryle, le monostéarate de glycéryle, le monooléate de glycéryle, le dicaprylate de glycéryle, le dilaurate de glycéryle, le myristate d'isopropyle, l'oléate d'isopropyle, le 2-pyrrolidone, le N-méthyl-2-pyrrolidone, la beta-cyclodextrine, la beta-cyclodextrine d'hydroxypropyle, le polyéthylèneglycol 200, le polyéthylèneglycol 300, le polyéthylèneglycol 400 et des mélanges de ceux-ci ; et
c) un ou plusieurs agent(s) tensio-actif(s) sélectionné(s) parmi le groupe qui est constitué par le monolaurate de sorbitane de PEG-20, le monopalmitate de sorbitane de PEG-20, le monostéarate de sorbitane de PEG-20, le monooléate de sorbitane de PEG-20, l'huile de castor hydrogénée de PEG-40, l'huile de castor de PEG-35, l'huile de noyau d'abricot de PEG-6 et des mélanges afférents.

2. Composition pharmaceutique selon la revendication 1, dans laquelle ladite composition est sous forme liquide, semi-solide ou solide.

3. Composition pharmaceutique selon la revendication 1 ou 2, dans laquelle :
a) le calanolide ou le mélange de calanolides est présent selon une quantité qui va de 0,5 % à 80 % en poids de la composition totale ;
b) l'agent favorisant la solubilité est présent selon une quantité qui va de 0,5 % à 90 % en poids de la composition totale ; et
c) l'agent tensio-actif est présent selon une quantité qui va de 0,1 % à 80 % en poids de la composition finale ;
ou
a) le calanolide ou le mélange de calanolides est présent selon une quantité qui va de 5 % à 50 % en poids de la composition totale ;
b) l'agent favorisant la solubilité est présent selon une quantité qui va de 10 % à 60 % en poids de la composition totale ; et
c) l'agent tensio-actif est présent selon une quantité qui va de 5 % à 50 % en poids de la composition finale ;
ou
a) le calanolide ou le mélange de calanolides est présent selon une quantité qui va de 10 % à 35 % en poids de la composition totale ;
b) l'agent favorisant la solubilité est présent selon une quantité qui va de 25 % à 50 % en poids de la composition totale ; et
c) l'agent tensio-actif est présent selon une quantité qui va de 20 % à 40 % en poids de la composition finale.

4. Composition pharmaceutique selon l'une quelconque des revendications précédentes, dans laquelle le calanolide est sélectionné parmi le groupe qui est constitué par les calanolides, leurs sels, esters, amides, isomères pharmaceutiquement acceptables, et leurs mélanges.

5. Composition pharmaceutique selon l'une quelconque des revendications précédentes, dans laquelle les particules de calanolide présentes dans la composition sont sous la forme cristalline, semi-cristalline, amorphe, semi-amorphe, liquide, solide dispersé, ou une/des combinaison(s) de celles-ci.

6. Composition pharmaceutique selon l'une quelconque des revendications précédentes, comprenant en outre au moins un excipient pharmaceutiquement acceptable.

7. Composition pharmaceutique selon l'une quelconque des revendications précédentes, dans laquelle ladite composition est présente selon une forme posologique unitaire pour une administration par voie orale ou parentérale.

8. Composition pharmaceutique selon l'une quelconque des revendications précédentes, comprenant en outre au moins un agent additionnel, dans laquelle l'agent additionnel est un agent anti-VIH, un agent anti-mycobactérien, ou une combinaison de ceux-ci.

9. Procédé de préparation de la composition pharmaceutique selon l'une quelconque des revendications précédentes, comprenant les étapes consistant à :
i) mélanger un calanolide ou un mélange de calanolides avec un ou plusieurs agent(s) favorisant la solubilité, tels que définis selon la revendication 1, à une température entre 20° C et 65 °C ;
ii) mélanger le mélange résultant obtenu au niveau de l'étape (i) avec un ou plusieurs agent(s) tensio-actif(s), tels que définis selon la revendication 1, de manière à former un mélange homogène ; et
dans lequel le procédé de mélange de l'étape (i) peut en outre comprendre une étape additionnelle consistant à broyer le mélange pulvérulent dans un broyeur à dispersion pendant 1 à 40 heure(s),
ou
i) mélanger un calanolide ou un mélange de calanolides selon une quantité de 0,5 % à 80 % en poids de la composition totale avec un ou plusieurs agent(s) favorisant la solubilité, tels que définis selon la revendication 1, selon une quantité de 0,5 % à 90 % en poids de la composition totale, à une température entre 20° C et 65 °C ;
ii) mélanger le mélange résultant obtenu au niveau de l'étape (i) avec un ou plusieurs agent(s) tensio-actif(s), tels que définis selon la revendication 1, selon une quantité de 0,1 % à 80 % en poids de manière à former un mélange homogène ; et
dans lequel le procédé de mélange de l'étape (i) peut en outre comprendre une étape additionnelle consistant à broyer le mélange pulvérulent dans un broyeur à dispersion pendant 1 à 40 heure(s),
ou
i) mélanger un calanolide ou un mélange de calanolides selon une quantité de 5 % à 50 % en poids de la composition totale avec un ou plusieurs agent(s) favorisant la solubilité, tels que définis selon la revendication 1, selon une quantité de 10 % à 60 % en poids de la composition totale, à une température entre 20° C et 65 °C ;
ii) mélanger le mélange résultant obtenu au niveau de l'étape (i) avec un ou plusieurs agent(s) tensio-actif(s), tels que définis selon la revendication 1, selon une quantité de 5 % à 50 % en poids de manière à former un mélange homogène ; et
dans lequel le procédé de mélange de l'étape (i) peut en outre comprendre une étape additionnelle consistant à broyer le mélange pulvérulent dans un broyeur à dispersion pendant 1 à 40 heure(s),
ou
i) mélanger un calanolide ou un mélange de calanolides selon une quantité de 10 % à 35 % en poids de la composition totale avec un ou plusieurs agent(s) favorisant la solubilité, tels que définis selon la revendication 1, selon une quantité de 25 % à 50 % en poids de la composition totale, à une température entre 20° C et 65 °C ;
ii) mélanger le mélange résultant obtenu au niveau de l'étape (i) avec un ou plusieurs agent(s) tensio-actif(s), tels que définis selon la revendication 1, selon une quantité de 20 % à 40 % en poids de manière à former un mélange homogène ; et
dans lequel le procédé de mélange de l'étape (i) peut en outre comprendre une étape additionnelle consistant à broyer le mélange pulvérulent dans un broyeur à dispersion pendant 1 à 40 heure(s).

10. Composition pharmaceutique selon l'une quelconque des revendications 1 à 8 pour une utilisation au niveau du traitement et de la prévention de maladies rétrovirales chez les mammifères.

11. Composition pharmaceutique selon l'une quelconque des revendications 1 à 8 pour une utilisation au niveau du traitement et de la prévention de maladies mycobactériennes chez les mammifères.

12. Composition pharmaceutique selon l'une quelconque des revendications 1 à 8 pour l'utilisation selon la revendication 10 ou 11, dans laquelle le mammifère est un être humain.
